# EUROPEAN PATENT APPLICATION

(11) **EP 4 249 499 A1**
(43) Date of publication of application: **27.09.2023**
(21) Application number: 21894792.7
(22) Date of filing: 06.05.2021
(51) Int. Cl.: C07K 14/00, C12N 15/70, A61P 31/14, A23L 33/17, G01N 33/569, A61K 47/42, A61K 38/00

(54) **CORONAVIRUS INFECTIOUS DISEASE COVID-19 THERAPEUTIC PROTEINS CTP ALPHA, CTP BETA, CTP GAMMA, CTP DELTA, AND USES THEREOF**

(30) Priority: 20.11.2020 KR 20200157143; 20.11.2020 KR 20200157147; 20.11.2020 KR 20200157148; 20.11.2020 KR 20200157149
(71) Applicant: Daegu Gyeongbuk Institute Of Science and Technology, Daegu 42988 (KR)
(72) Inventor: CHANG, Iksoo, Dalseong-gun Daegu 42988 (KR); KANG, Mooseok, Busan 47317 (KR); LEE, Ae, Ree, Dalseong-gun Daegu 42988 (KR); SEO, Souk, Dalseong-gun Daegu 42988 (KR); YU, Woo, Kyung, Daegu 42761 (KR); KWON, Wookbong, Daegu 41568 (KR); KIM, Hee, Yeon, Daegu 41148 (KR); PARK, Song, Dalseong-gun Daegu 42988 (KR); CHOI, Seong, Kyoon, Daegu 42116 (KR); KIM, Min, Gi, Namhae-gun Gyeongsangnam-do 52417 (KR); KIM, Sang, Yeol, Changwon-si Gyeongsangnam-do 51758 (KR); KIM, Hyo, Eun, Gyeongsan-si Gyeongsangbuk-do 38502 (KR); MIN, Ga, Hee, Dalseong-gun Daegu 42988 (KR); PARK, Seongjun, Changwon-si Gyeongsangnam-do 51142 (KR); LEE, Kyung, Eun, Dalseong-gun Daegu 42997 (KR); LEE, Juhwan, Changwon-si Gyeongsangnam-do 51621 (KR); JI, Sang, Ho, Dalseong-gun Daegu 42988 (KR); CHOI, Min, Jee, Ulsan 44073 (KR); CHOI, Jae, Seok, Dalseong-gun Daegu 43008 (KR); LEE, Young-Ho, Cheongju-si Chungcheongbuk-do 28115 (KR)
(74) Representative: Roos, Peter
(86) International application number: PCT/KR2021/005678
(87) International publication number: WO 2022/108013

(57) **Abstract**

The present invention relates to coronavirus infectious disease COVID-19 therapeutic proteins CTP alpha, CTP beta, CTP gamma, CTP delta, and uses thereof. Compared to a known peptide (P6) which mimics the binding site of a receptor binding domain (RBD) of SARS-CoV and angiotensin-converting enzyme 2 (ACE2), the therapeutic proteins CTP alpha, CTP beta, CTP gamma, and CTP delta according to the present invention comprise a novel part obtained by adding a novel sequence of amino acids, wherein the interaction of atoms constituting the amino acids has been fundamentally designed in order to strengthen the binding of SARS-CoV2 to a novel epitope of RBD. The present invention provides therapeutic proteins CTP alpha, CTP beta, CTP gamma, and CTP delta having a novel design and capable of binding more strongly than known peptides due to being creative designs of the extended therapeutic proteins CTP alpha, CTP beta, CTP gamma, and CTP delta, wherein the therapeutic proteins can additionally interact with charged amino acids of D420 and K458 on the rear side of the conventional binding interface between RBD and hACE2 or additionally interact with charged amino acids such as R454, K458, D467, and E471. The therapeutic proteins CTP alpha, CTP beta, CTP gamma, and CTP delta according to the present invention have the potential of being highly applicable as COVID-19 therapeutic agents.

## Description

### Technical Field

The present disclosure relates to coronavirus infectious disease COVID-19 therapeutic proteins CTP alpha (COVID-19 therapeutic protein alpha), CTP beta (COVID-19 therapeutic protein beta), CTP gamma (COVID-19 therapeutic protein gamma), and CTP delta (COVID-19 therapeutic protein delta), and uses thereof.

### Background Art

Coronavirus SARS-CoV2, which causes COVID-19 disease, binds to an angiotensin-converting enzyme 2 (hACE2) protein which is mainly present in human lung epithelial cells and invades into cells to replicate the viral genetic material in human cells to reproduce the virus. A fundamental way to treat COVID-19 infectious disease is to find strategies to prevent the coronavirus from entering human cells.

The spike protein S1-receptor binding domain (RBD) present on the surface of coronavirus binds to a hACE2 protein, wherein the 3D bound structure has been revealed by recent studies (Jun Lan, et al., Nature, 2020).

Cell invasion of coronavirus may be fundamentally deterred by blocking binding between RBD and hACE2, and it is possible to treat COVID-19 infectious disease by dramatically lowering the activity of coronavirus or eliminating infectivity thereof. Methods of neutralizing RBD of coronavirus is being actively studied as one of the principles of development for therapeutic agents.

The methods of neutralizing RBD are being studied to make other substances bind to RBD in advance instead of hACE2 so as to prevent RBD from binding to hACE2 on the cell surface. Developed or proposed as an RBD neutralizing therapeutic agent based on the existing hACE2 is human recombinant soluble ACE2 (hrsACE2=APN01) which is used as a lung cancer treatment. Also, idea-oriented peptide therapeutic agents have been proposed, most of which are designed based on mimicking only the binding site of hACE2. Accordingly, there is a need to develop protein therapeutic agents capable of binding to RBD more strongly than the previously presented peptide therapeutic agent.

### Disclosure of the Invention

### Technical Goals

The present disclosure relates to coronavirus infectious disease COVID-19 therapeutic proteins CTP alpha, CTP beta, CTP gamma, and CTP delta, and uses thereof, and an object of the present disclosure is to provide a protein which includes any one amino acid sequence selected from amino acid sequences represented by SEQ ID NO: 1 to SEQ ID NO: 10 and specifically binds to a receptor binding domain (RBD) of coronavirus, a composition for diagnosing coronavirus infection including the protein as an active ingredient, a pharmaceutical composition for preventing or treating coronavirus infectious disease, a health functional food composition for preventing or ameliorating coronavirus infectious disease, and a composition for drug delivery.

### Technical Solutions

In order to solve the above problems, the present disclosure provides a protein which includes any one amino acid sequence selected from amino acid sequences represented by SEQ ID NO: 1 to SEQ ID NO: 10 and specifically binds to a receptor binding domain (RBD) of coronavirus.

In addition, the present disclosure provides a polynucleotide encoding the protein, a recombinant vector including the polynucleotide, and a transformant (except for humans) transformed with the recombinant vector.

In addition, the present disclosure provides a composition for diagnosing coronavirus infection, including the protein as an active ingredient.

In addition, the present disclosure provides a pharmaceutical composition for preventing or treating coronavirus infectious disease, including the protein as an active ingredient.

In addition, the present disclosure provides a health functional food composition for preventing or ameliorating coronavirus infectious disease, including the protein as an active ingredient.

In addition, the present disclosure provides a composition for drug delivery, including the protein as an active ingredient.

### Advantageous Effects

The present disclosure relates to coronavirus infectious disease COVID-19 therapeutic proteins CTP alpha, CTP beta, CTP gamma, and CTP delta, and uses thereof, and, in order to make the binding of SARS-CoV2 RBa to a new epitope stronger, compared to a peptide (P6) mimicking the previously known binding site between SARS-CoV RBD and ACE2, the therapeutic proteins CTP alpha, CTP beta, CTP gamma, and CTP delta of the present disclosure include a new moiety added with a novel amino acid sequence that is fundamentally designed for interaction in the dimension of atoms consisting of the amino acids. Suggested in the present disclosure is a novel design of therapeutic proteins CTP alpha, CTP beta, CTP gamma, and CTP delta with stronger binding affinity than previously known peptides by uniquely designing an expanded therapeutic proteins CTP alpha, CTP beta, CTP gamma, and CTP delta capable of additionally interacting with charged amino acids of D420 and K458 located at the rear side of the previously known binding boundary between RBD and hACE2 or additionally interacting with charged amino acids such as R454, K458, D467, and E471, such that the therapeutic proteins CTP alpha, CTP beta, CTP gamma, and CTP delta of the present disclosure exhibit high applicability as a therapeutic agent for COVID-19.

### Brief Description of Drawings

FIG. 1 shows the binding structure of RBD (Pink) and CTP alpha.
FIG. 2 shows results of isolation and purification of therapeutic protein CTP-α1 expressed in E. coli. (A) CTP-α1 purified in BL21(DE3) RIL cells. M: Molecular weight markers, T: total cells after IPTG expression, S: aqueous solution after cell disruption and P: insoluble solution, Lane 1-10: a result of CTP-α1 purified by Ni-IMAC, (B) a result of CTP-α1 purified by ion exchange chromatography (IEX). (C) a result of SDS-PAGE for purely isolated and purified CTP-α1 having a dimer structure, and (D) a result of SDS-PAGE for finally purified CTP-α1 with 98% purity.
FIG. 3 shows results of isolation and purification of therapeutic protein CTP-α2 expressed in E. coli. (A) CTP-α2 purified in BL21 (DE3) RIL cells. M: Molecular weight markers, T: total cells after IPTG expression, S: aqueous solution after cell disruption, and P: insoluble solution, Lane 1-10: a result of CTP-α2 purified by Ni-IMAC, (B) a result of CTP-α2 purified by ion exchange chromatography (IEX). (C) a result of SDS-PAGE for purely isolated and purified CTP-α2 having a dimer structure, and (D) a result of SDS-PAGE for finally purified CTP-α2 with 99% purity.
FIG. 4 shows results of isolation and purification of therapeutic protein CTP-α3 expressed in E. coli. (A) CTP-α3 purified in BL21 (DE3) RIL cells. M: molecular weight markers, T: total cells after IPTG expression, S: aqueous solution after cell disruption, and P: insoluble solution, Lane 1-10: a result of CTP-α3 purified by Ni-IMAC, (B) a result of CTP-α3 purified by ion exchange chromatography (IEX). (C) a result of SDS-PAGE for purely isolated and purified CTP-α3 having a dimer structure, and (D) a result of SDS-PAGE for finally purified CTP-α3 with 95% purity.
FIG. 5 shows a result of the mass spectrometry of intact therapeutic protein CTP-α1.
FIG. 6 shows a result of the mass spectrometry of intact therapeutic protein CTP-α2.
FIG. 7 shows a result of the mass spectrometry of intact therapeutic protein CTP-α3.
FIG. 8 shows the sequence and MS/MS spectra of the N, C-terminus of therapeutic protein CTP-α1 identified using LC-MS/MS analysis.
FIG. 9 shows the sequence and MS/MS spectra of the N, C-terminus of therapeutic protein CTP-α2 identified using LC-MS/MS analysis.
FIG. 10 shows the sequence and MS/MS spectra of the N, C-terminus of therapeutic protein CTP-α3 identified using LC-MS/MS analysis.
FIG. 11 shows the far UV CD spectra of hACE2, RBD, and therapeutic protein CTP alpha.
FIG. 12 shows changes in the intensity of MST fluorescence measured by titrating hACE2 and CTP alpha in RBD.
FIG. 13 shows changes in cell viability by concentration upon treatment of therapeutic protein CTP-α1 to six cell lines HEK293T, HepG2, HMC-3, MRC-5, A549, and Caki-1 for 24 hours or 48 hours.
FIG. 14 shows changes in cell viability by concentration upon treatment of therapeutic protein CTP-α2 to six cell lines HEK293T, HepG2, HMC-3, MRC-5, A549, and Caki-1 for 24 hours or 48 hours.
FIG. 15 shows changes in cell viability by concentration upon treatment of therapeutic protein CTP-α3 to six cell lines HEK293T, HepG2, HMC-3, MRC-5, A549, and Caki-1 for 24 hours or 48 hours.
FIG. 16 shows the binding structure of RBD (Pink) and CTP beta.
FIG. 17 shows results of isolation and purification of therapeutic protein CTP-β1 expressed in E. coli. (A) CTP-β1 purified in BL21(DE3) RIL cells. M: Molecular weight markers, T: total cells after IPTG expression, S: aqueous solution after cell disruption and P: insoluble solution, Lane 1-10: a result of CTP-β1 purified by Ni-IMAC, (B) a result of CTP-β1 purified by ion exchange chromatography (IEX). (C) a result of SDS-PAGE for purely isolated and purified CTP-β1 having a dimer structure, and (D) a result of SDS-PAGE for finally purified CTP-β1 with 98% purity.
FIG. 18 shows results of isolation and purification of therapeutic protein CTP-β2 expressed in E. coli. (A) CTP-β2 purified in BL21 (DE3) RIL cells. M: Molecular weight markers, T: total cells after IPTG expression, S: aqueous solution after cell disruption and P: insoluble solution, Lane 1-10: a result of CTP-β2 purified by Ni-IMAC, (B) a result of CTP-β2 purified by ion exchange chromatography (IEX). (C) a result of SDS-PAGE for purely isolated and purified CTP-β2 having a dimer structure, and (D) a result of SDS-PAGE for finally purified CTP-β2 with 99% purity.
FIG. 19 shows results of isolation and purification of therapeutic protein CTP-β3 expressed in E. coli. (A) CTP-β3 purified in BL21 (DE3) RIL cells. M: Molecular weight markers, T: total cells after IPTG expression, S: aqueous solution after cell disruption and P: insoluble solution, Lane 1-10: a result of CTP-β3 purified by Ni-IMAC, (B) a result of CTP-β3 purified by ion exchange chromatography (IEX). (C) a result of SDS-PAGE for purely isolated and purified CTP-β3 having a dimer structure, and (D) a result of SDS-PAGE for finally purified CTP-β3 with 95% purity.
FIG. 20 shows a result of the mass spectrometry of intact therapeutic protein CTP-β1.
FIG. 21 shows a result of the mass spectrometry of intact therapeutic protein CTP-β2.
FIG. 22 shows a result of the mass spectrometry of intact therapeutic protein CTP-β3.
FIG. 23 shows the sequence and MS/MS spectra of the N, C-terminus of therapeutic protein CTP-β1 identified using LC-MS/MS analysis.
FIG. 24 shows the sequence and MS/MS spectra of the N, C-terminus of therapeutic protein CTP-β2 identified using LC-MS/MS analysis.
FIG. 25 shows the sequence and MS/MS spectra of the N, C-terminus of therapeutic protein CTP-β3 identified using LC-MS/MS analysis.
FIG. 26 shows the far UV CD spectra of hACE2, RBD, and therapeutic protein CTP beta.
FIG. 27 shows changes in the intensity of MST fluorescence measured by titrating hACE2 and CTP beta in RBD.
FIG. 28 shows changes in cell viability by concentration upon treatment of therapeutic protein CTP-β1 to six cell lines HEK293T, HepG2, HMC-3, MRC-5, A549, and Caki-1 for 24 hours or 48 hours.
FIG. 29 shows changes in cell viability by concentration upon treatment of therapeutic protein CTP-β2 to six cell lines HEK293T, HepG2, HMC-3, MRC-5, A549, and Caki-1 for 24 hours or 48 hours.
FIG. 30 shows changes in cell viability by concentration upon treatment of therapeutic protein CTP-β3 to six cell lines HEK293T, HepG2, HMC-3, MRC-5, A549, and Caki-1 for 24 hours or 48 hours.
FIG. 31 shows the binding structure of RBD (Pink) and CTP gamma.
FIG. 32 shows results of isolation and purification of therapeutic protein CTP-γ1 expressed in E. coli. (A) CTP-γ1 purified in BL21 (DE3) RIL cells. M: Molecular weight markers, T: total cells after expression of IPTG, S: aqueous solution after cell disruption and P: insoluble solution, Lane 1-10: a result of CTP-γ1 purified by Ni-IMAC, (B) a result of CTP-γ1 purified by ion exchange chromatography (IEX). (C) a result of SDS-PAGE for purely isolated and purified CTP-γ1 having a dimer structure, and (D) a result of SDS-PAGE for finally purified CTP-γ1 with 98% purity.
FIG. 33 shows results of isolation and purification of therapeutic protein CTP-γ2 expressed in E. coli. (A) CTP-γ2 purified in BL21 (DE3) RIL cells. M: Molecular weight markers, T: total cells after expression of IPTG, S: aqueous solution after cell disruption and P: insoluble solution, Lane 1-10: a result of CTP-γ2 purified by Ni-IMAC, (B) a result of CTP-γ2 purified by ion exchange chromatography (IEX). (C) a result of SDS-PAGE for purely isolated and purified CTP-γ2 having a dimer structure, and (D) a result of SDS-PAGE for finally purified CTP-γ2 with 99% purity.
FIG. 34 shows results of isolation and purification of therapeutic protein CTP-γ3 expressed in E. coli. (A) CTP-γ3 purified in BL21 (DE3) RIL cells. M: Molecular weight markers, T: total cells after expression of IPTG, S: aqueous solution after cell disruption and P: insoluble solution, Lane 1-10: a result of CTP-γ3 purified by Ni-IMAC, (B) a result of CTP-γ3 purified by ion exchange chromatography (IEX). (C) a result of SDS-PAGE for purely isolated and purified CTP-γ3 having a dimer structure, and (D) a result of SDS-PAGE for finally purified CTP-γ3 with 95% purity.
FIG. 35 shows a result of the mass spectrometry of intact therapeutic protein CTP-γ1.
FIG. 36 shows a result of the mass spectrometry of intact therapeutic protein CTP-y2.
FIG. 37 shows a result of the mass spectrometry of intact therapeutic protein CTP-γ3.
FIG. 38 shows the sequence and MS/MS spectra of the N, C-terminus of therapeutic protein CTP-γ1 identified using LC-MS/MS analysis.
FIG. 39 shows the sequence and MS/MS spectra of the N, C-terminus of therapeutic protein CTP-γ2 identified using LC-MS/MS analysis.
FIG. 40 shows the sequence and MS/MS spectra of the N, C-terminus of therapeutic protein CTP-γ3 identified using LC-MS/MS analysis.
FIG. 41 shows the far UV CD spectra of hACE2, RBD, and therapeutic protein CTP gamma.
FIG. 42 shows changes in the intensity of MST fluorescence measured by titrating hACE2 and CTP gamma in RBD.
FIG. 43 shows changes in cell viability by concentration upon treatment of therapeutic protein CTP-γ1 to six cell lines HEK293T, HepG2, HMC-3, MRC-5, A549, and Caki-1 for 24 hours or 48 hours.
FIG. 44 shows changes in cell viability by concentration upon treatment of therapeutic protein CTP-γ2 to six cell lines HEK293T, HepG2, HMC-3, MRC-5, A549, and Caki-1 for 24 hours or 48 hours.
FIG. 45 shows changes in cell viability by concentration upon treatment of therapeutic protein CTP-γ3 to six cell lines HEK293T, HepG2, HMC-3, MRC-5, A549, and Caki-1 for 24 hours or 48 hours.
FIG. 46 shows the binding structure of RBD (Pink) and CTP delta.
FIG. 47 shows results of isolation and purification of therapeutic protein CTP-61 expressed in E. coli. (A) CTP-δ1 purified in BL21 (DE3) RIL cells. M: Molecular weight markers, T: total cells after expression of IPTG, S: aqueous solution after cell disruption and P: insoluble solution, Lane 1-10: a result of CTP-δ1 purified by Ni-IMAC, (B) a result of CTP-δ1 purified by ion exchange chromatography (IEX). (C) a result of SDS-PAGE for purely isolated and purified CTP-δ1, and (D) a result of SDS-PAGE for finally purified CTP-δ1 with 98% purity.
FIG. 48 shows a result of the mass spectrometry of intact therapeutic protein CTP-δ1.
FIG. 49 shows the sequence and MS/MS spectra of the N, C-terminus of therapeutic protein CTP-61 identified using LC-MS/MS analysis.
FIG. 50 shows the far UV CD spectra of hACE2, RBD, and therapeutic protein CTP delta.
FIG. 51 shows changes in the intensity of MST fluorescence measured by titrating hACE2 and CTP delta in RBD.

### Best Mode for Carrying Out the Invention

The present disclosure provides a protein which includes any one amino acid sequence selected from amino acid sequences represented by SEQ ID NO: 1 to SEQ ID NO: 10 and specifically binds to a receptor binding domain (RBD) of coronavirus.

Specifically, the protein may inhibit binding between RBD of coronavirus and angiotensin-converting enzyme 2 (ACE2), but is not limited thereto.

Specifically, the coronavirus may be SARS-CoV2, but is not limited thereto.

Specifically, the protein which includes any one amino acid sequence selected from the amino acid sequences represented by SEQ ID NO: 1 to SEQ ID NO: 9 may bind to D420 and K458 of SARS-CoV2 RBD, and the protein which includes the amino acid sequence represented by SEQ ID NO: 10 may bind to R454, K458, D467, and E471 of SARS-CoV2 RBD, but is not limited thereto.

The protein of the present disclosure may be easily prepared by chemical synthesis known in the art (Creighton, Proteins; Structures and Molecular Principles, W. H. Freeman and Co., NY, 1983). Representative methods include liquid or solid phase synthesis, fragment condensation, and F-MOC or T-BOC chemical methods (Chemical Approaches to the Synthesis of Peptides and Proteins, Williams et al., Eds., CRC Press, Boca Raton Florida, 1997; A Practical Approach, Athert on & Sheppard, Eds., IRL Press, Oxford, England, 1989), but is not limited thereto.

In addition, the protein of the present disclosure may be prepared by a genetic engineering method. First, a DNA sequence encoding the protein is synthesized according to a conventional method. DNA sequences may be synthesized by PCR amplification using appropriate primers. DNA sequences may be synthesized by other standard methods known in the art, for example, using an automatic DNA synthesizer (e.g., those sold by Biosearch or Applied Biosystems). The prepared DNA sequence is inserted into a vector including one or more expression control sequences (e.g., promoter, enhancer, etc.) that are operatively linked to the DNA sequence to regulate the expression of the DNA sequence, and transform a host cell with the recombinant expression vector formed therefrom. The produced transformant is cultured under an appropriate medium and conditions to express the DNA sequence so as to harvest a substantially pure protein encoded by the DNA sequence from a culture. The harvest may be performed using a method known in the art (e.g., chromatography). The term 'substantially pure protein' as used herein refers to a state that the protein according to the present disclosure does not substantially include any other proteins derived from the host.

In the present disclosure, the protein including any one amino acid sequence selected from amino acid sequences represented by SEQ ID NO: 1 to SEQ ID NO: 10 is a concept including functional variants thereof. The term "functional variant" as used herein refers to all similar sequences in which some amino acid substitutions occur at amino acid loci that do not affect the properties of the protein of the present disclosure.

In addition, the present disclosure provides a polynucleotide encoding the protein.

The term "polynucleotide" as used herein refers to a polymer of deoxyribonucleotides or ribonucleotides that exist in single-stranded or double-stranded form. The polynucleotide includes RNA genomic sequences, DNA (gDNA and cDNA) and RNA sequences transcribed therefrom and also includes analogs of natural polynucleotides unless otherwise specified.

The polynucleotide includes not only the nucleotide sequence encoding the protein, but also a sequence complementary to the sequence. The complementary sequence includes not only perfectly complementary sequences, but also substantially complementary sequences.

In addition, the polynucleotide may be modified. The modifications include additions, deletions or non-conservative substitutions or conservative substitutions of nucleotides. The polynucleotide encoding the amino acid sequence is construed to include a nucleotide sequence showing substantial identity to the nucleotide sequence. The substantial identity may refer to, when the nucleotide sequence and any other sequence are aligned to the maximum correspondence and the aligned sequence is analyzed using an algorithm commonly used in the art, a sequence showing at least 80% homology, at least 90% homology or at least 95% homology.

In addition, the present disclosure provides a recombinant vector including the polynucleotide.

In addition, the present disclosure provides a transformant (except for humans) transformed with the recombinant vector.

The term "vector" as used herein refers to a self-replicating DNA molecule used to carry a clonal gene (or another fragment of clonal DNA).

The term "recombinant vector" as used herein may refer to a plasmid, viral vector or other media known in the art capable of expressing the inserted nucleic acid in a host cell, and one that polynucleotides encoding the protein of the present disclosure are operably linked to a conventional expression vector known in the art. The recombinant vector may include an origin of replication to generally enable proliferation in a host cell, and one or more expression regulatory sequences (e.g., promoter, enhancer, etc.) to regulate expression, a selective marker, and a polynucleotide encoding the protein of the present disclosure operably linked to an expression regulatory sequence. The transformant may be transformed by the recombinant vector.

Preferably, the transformant may be obtained by introducing a recombinant vector including a polynucleotide encoding the protein of the present disclosure into a host cell by a method known in the art, for example, but not limited to, transient transfection, microinjection, transduction, cell fusion, calcium phosphate precipitation, liposome-mediated transfection, DEAE dextran-mediated transfection, polybrene-mediated transfection, electroporation, gene gun, and other known methods for introducing a nucleic acid into the cell (Wu et al., J. Bio. Chem., 267:963-967, 1992; Wu and Wu, J. Bio. Chem., 263:14621-14624, 1988).

In addition, the present disclosure provides a composition for diagnosing coronavirus infection, including the protein as an active ingredient.

Preferably, the coronavirus may be SARS-CoV2, but is not limited thereto.

The term "diagnosis" as used herein refers to identification of the presence or characteristics of a pathological condition. For the purposes of the present disclosure, diagnosis is to identify the presence or characteristics of coronavirus infection.

Diagnosis of coronavirus infection using the protein of the present disclosure may be diagnosed by reacting the protein of the present disclosure with the tissue or cell obtained directly from blood, urine or biopsy, followed by detection of binding thereof.

In addition, in order to easily identify, detect and quantify whether the protein of the present disclosure binds to the RBD of coronavirus, the protein of the present disclosure may be provided in a labeled state. In other words, it may be provided by being linked (e.g., covalently bonded or cross-linked) to a detectable label. The detectable label may be a chromogenic enzyme (e.g., peroxidase, alkaline phosphatase), a radioactive isotope (e.g., ¹²⁴I, ¹²⁵I, ¹¹¹In, ⁹⁹mTc, ³²P, ³⁵S), a chromophore, a luminescent material or a fluorescent material (e.g., FITC, RITC, rhodamine, cyanine, Texas Red, fluorescein, phycoerythrin, and quantum dots).

Similarly, the detectable label may be an antibody epitope, a substrate, a cofactor, an inhibitor, or an affinity ligand. Such labeling may be performed during the process of synthesizing the protein of the present disclosure or may be additionally performed on the pre-synthesized protein. If a fluorescent substance is used as a detectable label, the coronavirus infection may be diagnosed by fluorescence mediated tomography (FMT). For example, the protein of the present disclosure labeled with the fluorescent substance may be circulated in the blood and the fluorescence by the protein may be observed by the fluorescence mediated tomography. If the fluorescence is detected, it is diagnosed as coronavirus infection.

In addition, the present disclosure provides a pharmaceutical composition for preventing or treating coronavirus infectious disease, including the protein as an active ingredient.

Preferably, the coronavirus infectious disease may be COVID-19, but is not limited thereto.

The pharmaceutical composition of the present disclosure may be prepared using a pharmaceutically suitable and physiologically acceptable adjuvant in addition to the active ingredient, and a solubilizing agent such as an excipient, a disintegrant, a sweetener, a binder, a coating agent, a swelling agent, a lubricant, a polishing agent, or a flavoring agent may be used as the adjuvant. The pharmaceutical composition of the present disclosure may be preferably formulated into a pharmaceutical composition by including one or more pharmaceutically acceptable carriers in addition to the active ingredient for administration. In the composition formulated into a liquid solution, pharmaceutically acceptable carriers may be sterile and biocompatible and used by mixing saline, sterile water, Ringer's solution, buffered saline, albumin injection, dextrose solution, maltodextrin solution, glycerol, ethanol and one or more of these components, while other conventional additives such as antioxidants, buffers, and bacteriostats may be added as needed. In addition, diluents, dispersants, surfactants, binders, and lubricants may be additionally added to formulate into an injectable formulation such as aqueous solutions, suspensions, and emulsions as well as pills, capsules, granules, or tablets.

The pharmaceutical formulation of the pharmaceutical composition of the present disclosure may be granules, powder, coated tablets, tablets, capsules, suppositories, syrups, juices, suspensions, emulsions, drops or injectable solutions, and sustained-release preparations of the active compound. The pharmaceutical composition of the present disclosure may be administered in a conventional manner via intravenous, intraarterial, intraperitoneal, intramuscular, intraarterial, intraperitoneal, intrasternal, transdermal, intranasal, inhalational, topical, rectal, oral, intraocular or intradermal routes. The effective amount of the active ingredient of the pharmaceutical composition of the present disclosure refers to an amount required for preventing or treating a disease. Therefore, the effective amount may be controlled by various factors including the type of disease, the severity of the disease, the type and content of the active ingredient and other ingredients included in the composition, the type of formulation and the age, weight, general health status, sex and diet of a patient, administration time, administration route and secretion rate of the composition, treatment period, and drugs used in combination with. For example, although not limited thereto, the composition of the present disclosure may be administered at a dose of 0.1 ng/kg to 10 g/kg when administered once to several times a day for adults.

In addition, the present disclosure provides a health functional food composition for preventing or ameliorating coronavirus infectious disease, including the protein as an active ingredient.

The health functional food composition of the present disclosure may be provided in the form of powder, granules, tablets, capsules, syrups or beverages, which may be used together with other foods or food additives in addition to the active ingredient and also be appropriately used according to a conventional method. The mixing amount of the active ingredient may be appropriately determined depending on the intended use thereof, for example, prophylactic, health, or therapeutic treatment.

The effective dose of the active ingredient included in the health functional food composition may be used in accordance with the effective dose of the pharmaceutical composition, but in the case of long-term intake for health and hygiene or health control, it should be less than or equal to the above range, and it is certain that the active ingredient may be used in an amount beyond the above range since there is no problem in terms of safety.

The type of health food is not particularly limited, and examples may include meat, sausage, bread, chocolate, candy, snacks, confectionery, pizza, ramen, other noodles, gum, dairy products including ice cream, various soups, beverages, tea, drinks, alcoholic beverages, vitamin complexes, and the like.

In addition, the present disclosure provides a composition for drug delivery, including the protein as an active ingredient.

Preferably, the drug may be an antiviral agent, and more preferably, the antiviral agent may be an antiviral agent against coronavirus which may be SARS-CoV2, but is not limited thereto.

The protein according to the present disclosure may be used as an intelligent drug carrier to selectively deliver drugs to coronavirus. If the protein of the present disclosure is used to treat coronavirus infectious disease by linking the protein with a conventionally known drug, the drug may be selectively delivered only to coronavirus by the protein of the present disclosure, such that the efficacy of the drug may be increased while the side effect of the drug is significantly reduced.

The drug is an antiviral agent, and as the antiviral agent that may be linked to the protein of the present disclosure, there is no limit as long as it is used for the conventional treatment of coronavirus infectious disease. The linkage between the antiviral agent and the protein of the present disclosure may be performed by methods known in the art, for example, covalent bonding, crosslinking, and the like. To this end, if necessary, the protein of the present disclosure may be chemically modified in a range in which the activity thereof is not lost.

### Modes for Carrying Out the Invention

Hereinafter, to help the understanding of the present disclosure, example embodiments will be described in detail. However, the following example embodiments are merely illustrative of the contents of the present disclosure, and the scope of the present disclosure is not limited to the following examples. The example embodiments of the present disclosure are provided to more completely explain the present disclosure to those of ordinary skill in the art.

### <Example 1> 3D structural analysis for a protein of coronavirus infectious disease COVID-19 therapeutic protein CTP alpha

### 1. Preparation of a 3D structure of a protein

A binding structure of hACE2-RBD registered in Protein Data Bank (PDB, https://www.rcsb.org/) (PDB ID: 6M0J) was used. The structure of a P6 moiety was modeled using MODELLER based on the binding structure of hACE2-RBD using the sequence of 22-44-G-351-357 of hACE2.

### 2. Molecular dynamic simulation

For the modeled hACE2-RBD complex, P6-RBD complex, CTP alpha monomer-RBD complex, CTP alpha dimer-RBD complex, P6, CTP alpha monomer, and CTP alpha dimer, molecular dynamics simulations to which the ff14SB force field was applied were performed using the AMBER18 package. The simulation was performed with the pmemd.cuda program included in AMBER18, and an octahedron TIP3P water box with a length of 15 Å added to the outer portion of the prepared protein was applied for the initial structure for the simulation. A periodic boundary condition was applied based on the water box, and Na+ and Cl- ions were added to neutralize the net charge. The particle-mesh Ewald (PME) method was applied for electromagnetic force of long distance based on a distance of 9 Å. The SHAKE algorithm to fix the distance of a covalent bond of hydrogen molecules was used, and the simulation was performed with a time step of 2 fs/step. First, a 5000-step minimization simulation was performed by applying 0.5 kcal/mol of a position restraint to the backbone structure of the protein, and a heating simulation was performed for 25 ps under NVT ensemble conditions from 10K to 300K by applying 0.1 kcal/mol of a position restraint. Equilibrium simulations were performed under NPT ensemble conditions at a temperature of 300K for 1 ns. The production run was performed under NPT ensemble conditions at a temperature of 300K and a pressure of 1 bar, and the simulation was performed for 500 ns for a complex with RBD and 1 µs for CTP alpha alone. Simulations were performed with 5 independent trajectories for each case.

The binding energy of RBD and CTP alpha were calculated by the MMGBSA algorithm using simulation snapshots within 300 to 500 ns, and entropy was calculated via normal mode analysis. The MMPBSA.py program in the AMBER18 package was used to calculate the binding free energy.

### 3. Structure of CTP alpha

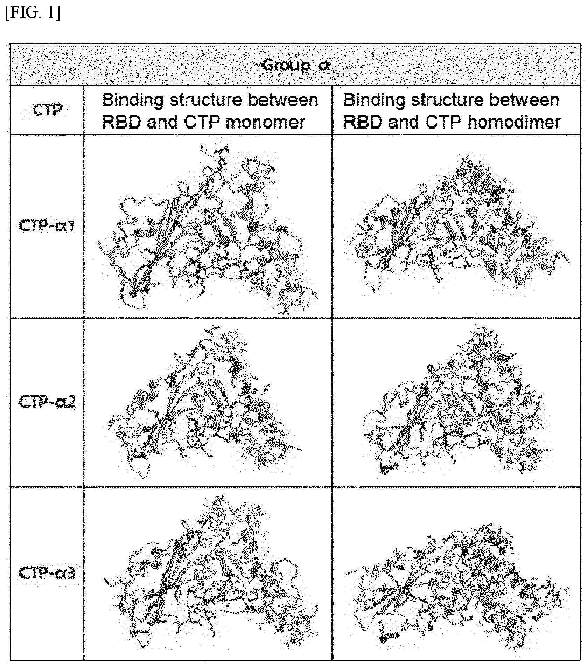

The modeled CTP alpha group (CTP-α1, CTP-α2, CTP-α3) has structures of an HTH secondarily improved type and a K2 structure reinforced type. When the structural ensemble of the modeled structure of the monomer and dimer forms in a solution was analyzed through molecular dynamics simulation, the structural patterns shown in FIG. 1 were observed.

CTP-α1 is a therapeutic protein with K2 subjected to interaction with D420 of RBD as well as E4 subjected to interaction with K458 positioned at the N-terminal portion. It is a therapeutic protein with improved stability in the secondary structure.

CTP-α2 is a therapeutic protein with K2 subjected to interaction with D420 of RBD as well as D4 subjected to interaction with K458 positioned at the N-terminal portion. It is a therapeutic protein with improved stability in the secondary structure.

CTP-α3 is a therapeutic protein with K2 subjected to interaction with D420 of RBD as well as E7 subjected to interaction with K458 positioned at the N-terminal portion. It is a therapeutic protein with improved stability in the secondary structure.

### 4. Prediction of binding free energy of CTP alpha-RBD

Binding energy and binding free energy were calculated to determine how well CTP alpha binds to RBD (Table 1). Using molecular dynamics simulation, structural ensembles in which hACE2, P6, and CTP alphas bind to RBD, respectively, were collected, and the binding energy (ΔE) and binding free energy (ΔG) from the structural ensemble were analyzed using the MMGBSA algorithm.

It was found that CTPs have negative binding free energy and are stable when bound to RBD, bind with stronger energy (ΔE) than hACE2 and P6 in simulations due to interaction with a new epitope, and also bind with similar or stronger free energy (ΔG).

**TABLE 1**

| Group | CTP | RBD + CTP monomer | | RBD + CTP dimer | |
|---|---|---|---|---|---|
| | | ΔE | ΔG | ΔE | ΔG |
| - | hACE2 | -74.0327 | - | - | - |
| - | P6 | -63.9558 | -11.0060 | - | - |
| CTP α | CTP-α1 | -65.0212 | -11.5714 | -76.1909 | -11.8224 |
| | CTP-α2 | -81.5078 | -20.3598 | -85.9801 | -13.2608 |
| | CTP-α3 | -66.9183 | -11.1192 | -64.5851 | -7.4985 |

### 5. In-silico immunogenicity analysis of CTP alpha

In-silico immunogenicity was analyzed using the globally well-established NetMHC-4.0 server (http://www.cbs.dtu.dk/services/NetMHC/). For the amino acid sequence of CTP alpha, prediction was made on the binding of peptide-MHC class I for 81 MHC alleles of each different individual (human) and 41 alleles of animals (monkey, cattle, pig, and mouse) in the NetMHC4.0 server. By searching in peptide units of 13-14 amino acids in length, the total number of searched peptides and the number of peptides expected to have strong binding (SB) and weak binding (WB) thereamong were counted, and the ratio was calculated by (SB+WB)/Total to verify immunogenicity.

It indicates that the lower the ratio to the total (SB+WB)/Total is, the less the antigen-antibody reaction occurs with other proteins when the neutralizing therapeutic protein of the present disclosure is injected into the body (Tables 2 to 7).

**TABLE 2**

| Human | | | | | | |
|---|---|---|---|---|---|---|
| Group | CTP | scan length | Total | SB | WB | (SB+WB)/T otal |
| CTP α | CTP-α1 | 13mer | 3402 | 6 | 23 | 0.009 |
| | | 14mer | 3321 | 10 | 29 | 0.012 |
| | CTP-α2 | 13mer | 3402 | 7 | 19 | 0.008 |
| | | 14mer | 3321 | 10 | 25 | 0.011 |
| | CTP-α3 | 13mer | 3402 | 6 | 26 | 0.009 |
| | | 14mer | 3321 | 10 | 32 | 0.013 |

**TABLE 3**

| Chimpanzee | | | | | | |
|---|---|---|---|---|---|---|
| Group | CTP | scan length | Total | SB | WB | (SB+WB)/T otal |
| CTP α | CTP-α1 | 13mer | 336 | 1 | 1 | 0.006 |
| | | 14mer | 328 | 0 | 4 | 0.012 |
| | CTP-α2 | 13mer | 336 | 0 | 2 | 0.006 |
| | | 14mer | 328 | 0 | 4 | 0.012 |
| | CTP-α3 | 13mer | 336 | 1 | 1 | 0.006 |
| | | 14mer | 328 | 0 | 4 | 0.012 |

**TABLE 4**

| Rhesus macaque | | | | | | |
|---|---|---|---|---|---|---|
| Group | CTP | scan length | Total | SB | WB | (SB+WB)/T otal |
| CTP α | CTP-α1 | 13mer | 756 | 4 | 22 | 0.034 |
| | | 14mer | 738 | 6 | 29 | 0.047 |
| | CTP-α2 | 13mer | 756 | 4 | 18 | 0.029 |
| | | 14mer | 738 | 6 | 26 | 0.043 |
| | CTP-α3 | 13mer | 756 | 4 | 17 | 0.028 |
| | | 14mer | 738 | 6 | 25 | 0.042 |

**TABLE 5**

| Mouse | | | | | | |
|---|---|---|---|---|---|---|
| Group | CTP | scan length | Total | SB | WB | (SB+WB)/T otal |
| CTP α | CTP-α1 | 13mer | 252 | 1 | 13 | 0.056 |
| | | 14mer | 246 | 1 | 17 | 0.073 |
| | CTP-α2 | 13mer | 252 | 1 | 11 | 0.048 |
| | | 14mer | 246 | 1 | 14 | 0.061 |
| | CTP-α3 | 13mer | 252 | 1 | 10 | 0.044 |
| | | 14mer | 246 | 1 | 13 | 0.057 |

**TABLE 6**

| BoLA | | | | | | |
|---|---|---|---|---|---|---|
| Group | CTP | scan length | Total | SB | WB | (SB+WB)/T otal |
| CTP α | CTP-α1 | 13mer | 252 | 0 | 0 | 0.000 |
| | | 14mer | 246 | 0 | 0 | 0.000 |
| | CTP-α2 | 13mer | 252 | 0 | 0 | 0.000 |
| | | 14mer | 246 | 0 | 0 | 0.000 |
| | CTP-α3 | 13mer | 252 | 0 | 0 | 0.000 |
| | | 14mer | 246 | 0 | 0 | 0.000 |

**TABLE 7**

| Pig | | | | | | |
|---|---|---|---|---|---|---|
| Group | CTP | scan length | Total | SB | WB | (SB+WB)/T otal |
| CTP α | CTP-α1 | 13mer | 126 | 0 | 0 | 0.000 |
| | | 14mer | 123 | 0 | 1 | 0.008 |
| | CTP-α2 | 13mer | 126 | 0 | 0 | 0.000 |
| | | 14mer | 123 | 0 | 1 | 0.008 |
| | CTP-α3 | 13mer | 126 | 0 | 0 | 0.000 |
| | | 14mer | 123 | 0 | 1 | 0.008 |

### <Example 2> Cloning of coronavirus infectious disease COVID-19 therapeutic protein CTP alpha

### 1. Strain and medium

All chemicals used for gene cloning were of analytical grade. Completed clones were transformed using a DH5α E. coli strain for proliferation and screened with LB medium in which ampicillin (LPS, 100 µg/ml) was added. Cell culture was performed at 37°C with stirring involved.

### 2. Construction of plasmids

After converting the amino acid sequence of the protein designed in silico into nucleotides using the Sequence Manipulation Suite (https://www.bioinformatics.org/sms2/rev_trans.html) tool, DNA sequences were determined by comparing with the sequence present in hACE2. The synthesized DNA fragment (Bioneer, gene synthesis) was amplified by PCR using a forward primer 5'-GGAGATATACATATGAAAAGTGAACTTGCTTCTAATGTG(CTP-α1), 5'-GGAGATATACATATGAAAAGTGATCTTGCTTCTAATGTGT(CTP-α2), and 5'-GGAGATATACATATGAAAAGTCAACTTGCTGAAAATGTGT(CTP-α3) as well as a common reverse primer 5'-GTGGTGCTCGAGCCTGAAGTCGCCCTTCC, and then inserted into a pET-21a vector treated with restriction enzymes Nde I and Xho I by ligation independent cloning using EZ-Fusion^{™} HT Cloning Kit (Engnomics).

### 3. Experiment result

The therapeutic protein CTP alpha of the present disclosure is a therapeutic protein of a new amino acid sequence that neutralizes or inhibits the S1-RBD protein from binding to the hACE2 protein, wherein the amino acid sequence of the therapeutic protein CTP alpha designed in silico is shown in Table 8.

**TABLE 8**

| Group α | |
|---|---|
| CTP name | Sequence |
| CTP-α1 | MKSELASNVYNTNITKENVQNMNEEQAKTFLDKFNHEAEDLFY QSSGLGKGDFR (SEQ ID NO: 1) |
| CTP-α2 | MKSDLASNVYNTNITKENVQNMNEEQAKTFLDKFNHEAEDLFY QSSGLGKGDFR (SEQ ID NO: 2) |
| CTP-α3 | MKSQLAENVYNTNITKENVQNMNEEQAKTFLDKFNHEAEDLFY QSSGLGKGDFR (SEQ ID NO: 3) |

### <Example 3> Expression and purification of coronavirus infectious disease COVID-19 therapeutic protein CTP alpha

### 1. Protein expression and purification materials

All chemicals used for protein expression and purification were of analytical grade. Ampicillin, 1-thio-β-d-galactopyranoside (IPTG), sodium phosphate-monobasic, sodium phosphate-dibasic, and TRIS used for protein expression and purification were purchased from LPS (Daejeon, Korea), Coomassie brilliant blue R-250, 2-mercaptoethanol, chloramphenicol, and imidazole were purchased from Biosesang (Seongnam, Korea), sodium chloride was purchased from Duksan (Daejeon, Korea), and all columns were purchased from GE Healthcare (Piscataway, NJ). Phenylmethanesulfonyl fluoride (PMSF), trizma hydrochloride, and Amicon Ultra-15 Centrifugal Filter Units used herein were purchased from Millipore (Billerica, MA).

### 2. Protein expression and purification instruments

Shake incubator (NBS/Innova 42R) and high-performance high-capacity centrifuge (Beckman Coulter. Inc./AVANTI JXN-26) were used for protein expression, and protein high-speed separation system (GE Healthcare/AKTA Pure M and Start System), protein high-speed separation system (GE HealthcareIFPLC Accessory System), ultrasonicator (Q700-Sonicator), and refrigerated centrifuge (Epoendorf-5810R) were used to isolate and purify proteins.

### 3. Expression and culture of therapeutic protein CTP alpha

The gene cloned in the pET-21a vector was used for transformation for expression using a BL21 (DE3) RIL E. coli strain, and cell culture was performed at 37°C using LB medium in which ampicillin (100 mg/ml) and chloramphenicol (50 mg/ml) were added. After culture to meet OD₆₀₀ value of 0.6-0.8 (OD₆₀₀=0.6~0.8), 1M IPTG was added to a final concentration of 0.5 mM, followed by expression at 37°C for 4 hours. A high-performance high-capacity centrifuge (Beckman Coulter. Inc./AVANTI JXN-26) set at 7000 RPM, 4°C was used to harvest the cells for 20 minutes to be used immediately or stored at -80°C.

### 4. Isolation and purification of therapeutic protein CTP alpha

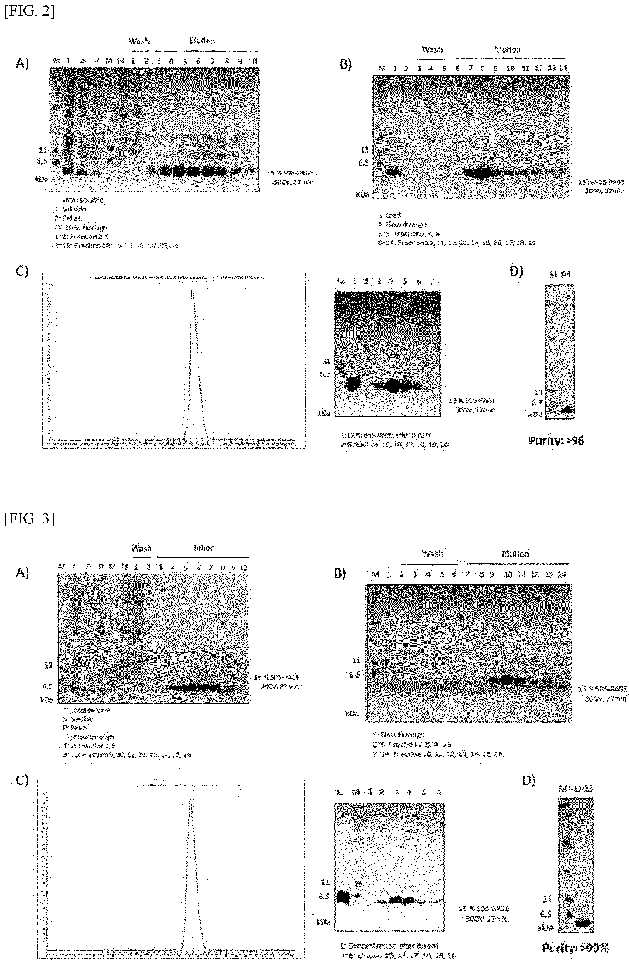

After dissolving in 10 ml of lysis buffer (pH 8.0, 50 mM Tris-HCl, 500 mM NaCl, 1 mM PMSF, 0.25% Tween-20 (v/v)) based on 1 g of cells, the cells were disrupted using a sonicator (Q700-Sonicator). In order to isolate the solution and the pellet, a high-speed centrifuge set at 18000 RPM and 4°C was used for 30 minutes. A 0.45 µm syringe filter was used for removal of impurities in the aqueous solution. The solution from which impurities were removed was poured through a HisTrap HP 5ml (GE Healthcare) column stabilized with wash buffer (pH 8.0, 50 mM Tris-HCl, 500 mM NaCl), and then elution buffer (pH 8.0, 50 mM Tris-HCl, 500 mM NaCl, 500 mM imidazole) was set to 0 to 100% gradient elution. As a result, it was found that the therapeutic protein CTP alpha was isolated with imidazole at a concentration of 100 to 300 mM. The isolated therapeutic protein CTP alpha was subjected to buffer exchange (pH 8.0, 20 mM Tris-HCl) in a cold room for 15 hours. After pouring the NaCl-removed CTP alpha solution through the HiTrap Q HP 5ml (GE Healthcare) column stabilized with binding buffer (pH 8.0, 20 mM Tris-HCl), elution buffer (pH 8.0, 20 mM Tris-HCl, 1.0 M NaCl) was set from 0 to 100% gradient elution. As a result, it was found that the therapeutic protein CTP alpha was isolated with NaCl at a concentration of 120 to 250 mM. Finally, therapeutic protein CTP alpha having a dimer structure was purely isolated and purified using a HiLoad Superdex 75 16/600 column stabilized with pH 7.4, 10 mM phosphate and 150 mM NaCl. Quantification was performed at A₂₈₀ₙₘ using NanoDrop, and, as a result of calculating the purity using the ImageJ program, high purity (>95%) therapeutic protein CTP alpha protein was obtained (FIGS. 2 to 4).

### <Example 4> Mass spectrometry of coronavirus infectious disease COVID-19 therapeutic protein CTP alpha

### 1. Mass spectrometry analysis

Mass spectrometry was used to identify the total mass and information on the amino acid sequence of the protein. Mass spectrometry of total proteins was performed using a high-performance matrix laser desorption ionization mass spectrometer (Bruker, UltrafleXtreme MALDI TOF/TOF). Hybrid triple quadrupole linear ion trap mass spectrometer (Sciex, QTRAP) and quadrupole orbitrap mass spectrometer (Thermo scientific, Q Exactive PLUS) were used for amino acid sequence analysis for the protein and micro liquid chromatography (Sciex, M5 MicroLC) was used for QTRAP, and nano-micro liquid chromatography (Waters, ACQUITY UPLC M-Class system) was used for Q Exactive PLUS.

### 2. Sample preparation for MALDI TOF and analysis of intact proteins using MALDI TOF

For total mass spectrometry of ACE2, RBD, and CTP alpha proteins, 10 µg of protein obtained by removing low-molecular compounds using Microcon centrifugal filter (Merck, MRCPRT010) and sinapinic acid (Bruker, 8201345) matrix were mixed in a 1:1 ratio, and the mixture was then placed on a target plate to dry and subjected to the flexcontrol program of the high-performance matrix laser desorption ionization mass spectrometer. The mass value was normalized using the Starter kit for MALDI-TOF MS (Bruker, 8208241), and the spectrum was obtained with an average of 1000 laser shots based on the method provided by flexcontrol. The result values were derived from each spectral data using the flexanalysis program.

### 3. Sample preparation for LC-MS/MS analysis

For amino acid sequence analysis of a single protein, 20 µg of RBD, hACE2, and CTP alpha proteins were mixed with 100 mM triethylammonium bicarbonate (Sigma, T7408-100ML) and 2% sodium dodecyl sulfate (Duksan, 6645) to a specific concentration. After adding 10 mM dithiothreitol (BIO-RAD, 161-0611) and treating at 56°C for 30 minutes, 20 mM iodoaetamide (Sigma, T1503) was added and shielded from light, followed by treatment at room temperature for 30 minutes. Then, using S-trap mini columns (PROTIFI, C02-mini-80), low molecular weight compounds were removed, and 2 µg of proteases including trypsin (Promega, V5111) and Glu-C (Promega, V1651) were added, followed by enzymatic degradation at 37°C for 16 hours. After termination of the reaction, isolation was performed with a centrifuge, drying was followed under vacuum, and 0.1% trifluoroacetic acid was added to prepare a sample. In addition, deglycosylation experiments were added for RBD and ACE2 proteins. For deglycosylation experiment, 100 mM triethylammonium bicarbonate and 2 µg of PNGase F (Promega, V4831) were added to the protease-treated sample, followed by enzymatic degradation at 37°C for 4 hours. After termination of the reaction, the proteins were isolated by centrifugation using S-trap mini columns. Vacuum drying was followed, and then 0.1% trifluoroacetic acid was added to prepare a sample. For the RBD and hACE2 proteins, a quadrupole orbitrap mass spectrometer was used, and experiments proceeded via nano-micro liquid chromatography. For the CTP alpha protein, a hybrid triple quadrupole linear ion trap mass spectrometer was used, and experiments proceeded using micro-liquid chromatography.

### 4. LC-MS/MS Analysis: ACQUITY UPLC M-Class system - Q Exactive PLUS

All solvents used for liquid chromatography were of HPLC grade. Water (Duksan, 7732-18-5) and acetonitrile (Honeywell, AH015-4) were used as solvents, and dimethyl sulfoxide (Sigma, 472301-1L), trifluoroacetic acid (Thermo scientific, 28904), and formic acid (Thermo scientific, 28905) were used as a medium in the experimental solution. Nano-micro liquid chromatography was composed of solvent A (0.1% formic acid, 5% dimethyl sulfoxide in water) and solvent B (0.1% formic acid, 5% dimethyl sulfoxide in 80% acetonitrile). Acclaim^{™} PepMap^{™} 100 C18 LC Column (Thermo scientific, 164197) was used as a trapping column, and EASY-Spray^{™} HPLC Columns (Thermo scientific, ES803A) as an analytical column. Liquid chromatography was performed for 5 minutes in the presence of 95% solvent A and 5% solvent B at a flow rate of 8 µl/min in the trapping step after injecting 0.5 µg of the sample. In the analytical step, the flow rate was 250 nl/min. After 5 minutes under the conditions of 95% solvent A and 5% solvent B, the samples were analyzed by gradually increasing the rate of solvent B from 5% to 40% for 140 minutes, the column was activated for 15 minutes after increasing to 95% for 5 minutes again, and re-equilibration was followed by decreasing the rate of solvent B to 5% for 5 minutes and maintaining the state for 40 minutes. The mass values of quadrupole orbitrap mass spectrometer were calibrated by Pierce^{™} LTQ Velos ESI Positive Ion Calibration Solution (Thermo scientific, 88323). The analysis was conducted via full-MS and data dependent MS/MS methods. The calculated data was obtained using a proteome discoverer (Thermo scientific).

### 5. LC-MS/MS Analysis: M5 MicroLC - QTRAP

The column used for the analysis was a Kinetex 2.6u XB-C18 100A (Phenomenex, 00B-4496-AC). Micro liquid chromatography was composed of solvent A (0.1% formic acid in water) and solvent B (0.1% formic acid in acetonitrile). The liquid chromatography was performed at a flow rate of 20 µl/min after injecting 1.5 µg of the sample. After 5 minutes under the conditions of 95% solvent A and 5% solvent B, the samples were analyzed by gradually increasing the rate of solvent B from 5% to 40% for 45 minutes, the column was activated for 4 minutes after increasing to 95% for 5 minutes again, and re-equilibration was followed by decreasing the rate of solvent B to 5% for 3 minutes and maintaining the state for 3 minutes. The mass values of hybrid triple quadrupole linear ion trap mass spectrometer were calibrated by standard chemical kit (Sciex, 4406127). The analysis was conducted using Q3 MS and enhanced product ion method using information dependent acquisition. For the calculated data, the wiff file was converted to the mgf file using convert MS (proteowizard.sourceforge.net), and the results were obtained using searchGUI and peptide-shaker (compomics.github.io).

### 6. Results of intact protein mass spectrometry

The mass of the intact protein was measured using MALDI-TOF. The mass of RBD was 30 kDa. In addition to 30kDa, the peak was a dimer, appeared along with a 60kDa peak, a monomer with a charge (z) of 2 (m/z=15kDa), and a trimer with a charge (z) of 2 (m/z=45kDa). Considering that the mass of RBD calculated from the protein sequence is 25.9 kDa, it may be suggested that glycosylation caused the change in mass of 4.1 kDa. Similarly, the mass of hACE2 was measured to be about 102 kDa, and a dimer and a peak with a charge of 2 also appeared together. Considering that the mass of hACE2 calculated from the protein sequence is 84.4 kDa, it is suggested that the mass change of about 17.5 kDa was caused by glycosylation.

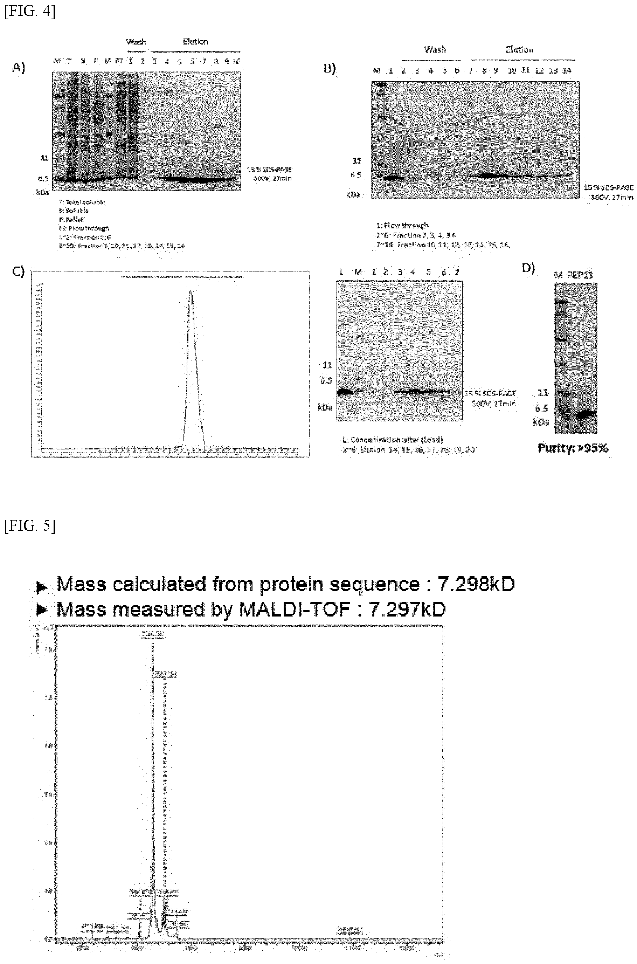

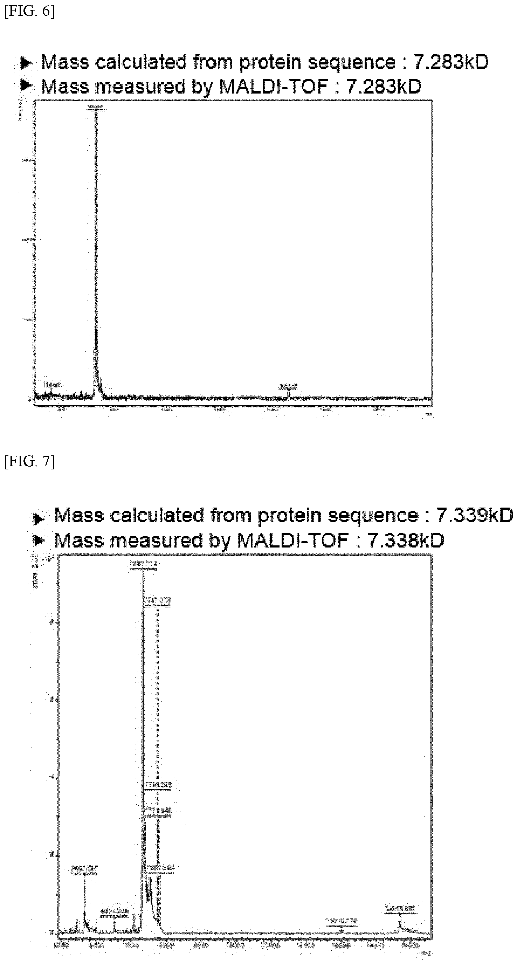

CTP-α1 was measured to be 7.297 kD, almost equal to the mass calculated from the sequence (FIG. 5), CTP-α2 was measured to be 7.283 kD, almost equal to the mass calculated from the sequence (FIG. 6), and CTP-α3 was measured to be 7.338 kD, almost equal to the mass calculated from the sequence (FIG. 7).

### 7. Identification of protein sequences

LC-MS/MS analysis was performed to identify the sequences of RBD, hACE2, and CTP alpha. Each protein was subjected to protein analysis using proteases such as trypsin and Glu-C, wherein, for RBD and hACE2, N-glycosylated Asn was found by searching for amino acid residues in which Asn was changed to Asp by enzymatic activity of PNGase F.

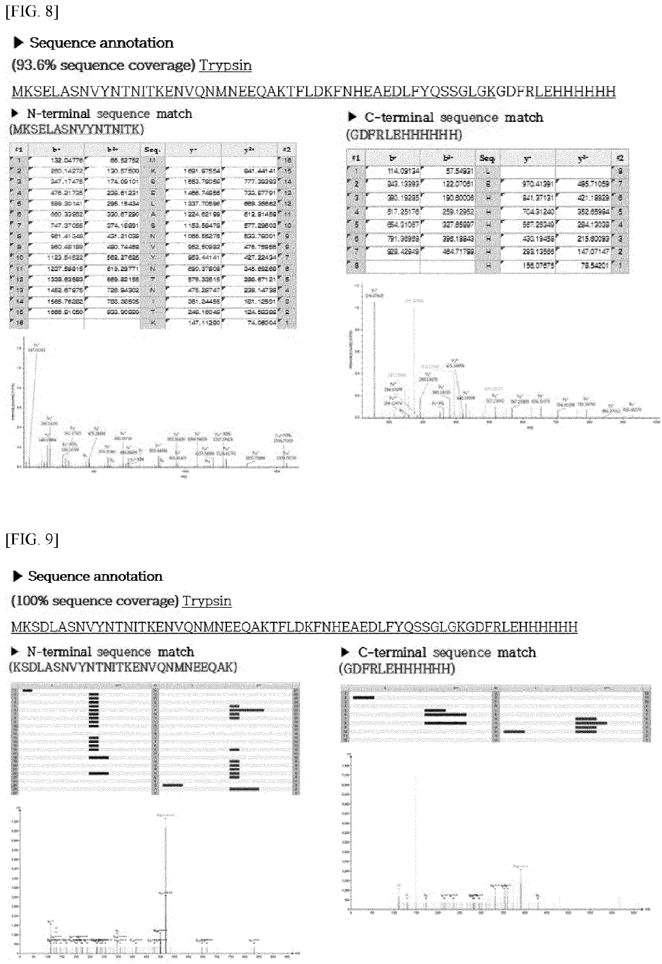

The results of MS/MS analysis of CTP alpha having the N, C-terminus identified are shown in FIGS. 8 to 10. Sequence analysis of the N, C-terminus is one of the means for identifying the state of the protein, and if the terminus of the protein is not identified, it may be considered as being degraded due to a problem in the preservation state of the protein. In the case of RBD and hACE2, the terminus of the proteins tends not to be identified (ID), so unless inaccuracy in the sequence or the effect of PTM matters, they are easily degraded, so care must be taken for preservation.

### <Example 5> Secondary structural stability and function/efficacy verification of coronavirus infectious disease COVID-19 therapeutic protein CTP alpha

### 1. Circular dichroism (CD) experiment

Far UV circular dichroism was measured for RBD (PBS buffer), hACE2 (PBS buffer), and CTP alpha. A JASCO-1500 machine was used with the protein concentration of 0.2 mg/ml and a quartz cell of 0.1 cm at a temperature of 20°C. Measurements were performed at a measurement speed of 20 nm/min, a bandwidth of 5 nm, and a digital integration time (D.I.T) of 4 seconds at a far UV wavelength of 190-250 nm. A graph was drawn by subtracting the data obtained by 5 times measurement for the protein by the data obtained by 5 times measurement for the buffer.

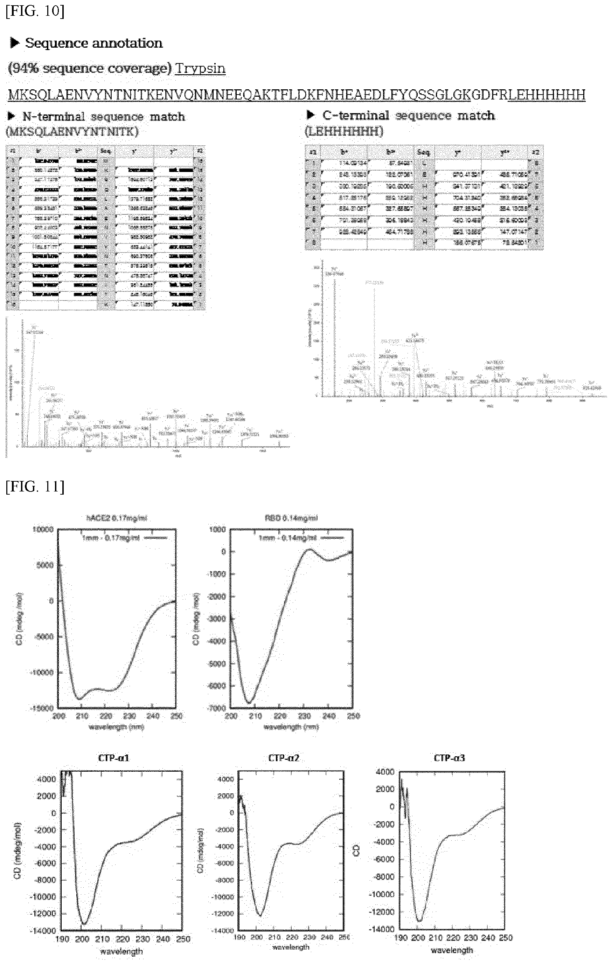

As a result, it was found that hACE2 is a protein having α-helix as a secondary structure, and RBD is a protein having a beta sheet as a secondary structure. It was found that CTP alpha has both a helical structure and a coil structure (FIG. 11).

### 2. Microscale thermophoresis (MST) experiment

Fluorescence was attached to 100 µL of 8 µM RBD (PBS buffer with 0.05% Tween-20 added) using Monolith Protein Labeling Kit RED-NHS 2nd Generation. Ligand (hACE2 (PBS buffer) and CTP alpha were prepared by 10 µL each with 16 concentrations from 16 µM to 0.488 nM using a 1:1 serial dilution. A mixed solution of 15 nM RBD and 8 µM-0.244 nM ligand was prepared by diluting the fluorescence-attached RBD to 30 nM and adding 10 µL of the RBD to each ligand of 16 concentrations. MST was measured 5 times for each ligand using a Monolith NT.115 machine with 60% excitation power and medium MST power. Kd (dissociation constant) value was calculated using Kdmodel of MO affinity analysis program provided by Monolith company. Experimental results having the median Kd value derived from 5 experiments are shown in a graph.

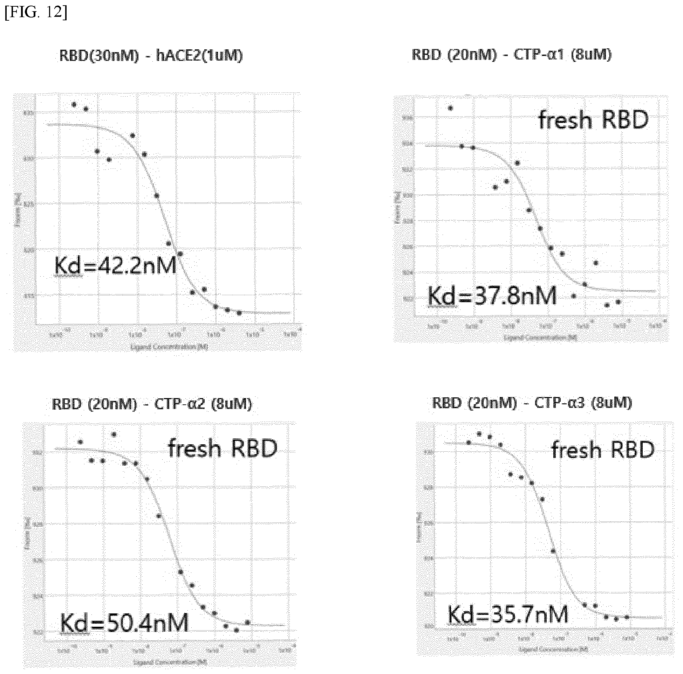

As a result, a change in concentration-dependent fluorescence intensity was observed, indicating that both hACE2 and CTP alpha bind to RBD. The Kd values representing the binding strength were 37.8 nM for CTP-α1, 50.4 nM for CTP-α2, 35.7 nM for CTP-α3, and 42.2 nM for hACE2 (FIG. 12). The Kd values of RBD and hACE2 are similar to that of 34.6 nM measured by using BLI (biolayer interferometry) and SPR (Surface Plasmon Resonance) in the Science paper (Wrapp et al, Science 367, 1260-1263(2020)).

### <Example 6> Toxicity test for coronavirus infectious disease COVID-19 therapeutic protein CTP alpha

### 1. Cell culture for toxicity analysis of therapeutic protein CTP alpha

The cell line used in the present disclosure was obtained from the Korea Cell Line Bank (KCLB, Seoul, Korea). Used as a culture medium for the human embryonic kidney cell line HEK293T, the human liver cancer cell line HepG2, and the human lung cancer cell line A549 was Dulbecco's modified eagle medium (DMEM, Hyclone, USA) supplemented with 10% fetal bovine serum (FBS, Hyclone, USA) and 1% antibiotic (penicillin-streptomysin; P/S, Gibco, USA), and human microglia HMC-3 was cultured in Minimum Essential Media (MEM, Gibco, USA) supplemented with 10% fetal bovine serum and 1% antibiotic (penicillin-streptomycin). Used as a culture medium for human normal lung cell line MRC-5 was Minimum Essential Media (MEM) supplemented with 10% fetal bovine serum, 1% antibiotic (penicillin-streptomycin) and 25 µM hydroxyethyl piperazine ethane sulfonic acid (HEPES, Hyclone, USA). For the human kidney cancer cell line Caki-1, McCoy's 5A medium (Gibco, USA) supplemented with 10% fetal bovine serum and 1% antibiotics (penicillin-streptomycin) was used as a culture medium. All cell lines used were cultured in an incubator controlled to a temperature of 37°C in the presence of 5% CO₂.

### 2. Cytotoxicity analysis for therapeutic protein CTP alpha

The HEK293T, HepG2, HMC-3, MRC-5, A549, and Caki-1 cell lines were dispensed into 96-well cell culture plates in the count of 3×10³, 6×10³, 5×10³, 6×10³, 5×10³, and 5×10³ cells/well, respectively, and then stabilized in an incubator controlled at a temperature of 37°C in the presence of 5% CO₂ for 24 hours. Then, a starvation culture medium in which the FBS content was reduced from 10% to 1% in the culture medium composition for each cell line was prepared and replaced with a starvation culture medium, followed by starvation for 16 hours. Thereafter, 1 fg, 10 fg, 100 fg, 1 pg, 10 pg, 100 pg, 1 ng, 10 ng, 100 ng, 1 µg, and 10 µg of CTP alpha were added to 100 µl of the culture medium, respectively, and the mixture was treated to the cell line, followed by culture for 24 hours and 48 hours. Viability of the cell lines was measured using Cell Counting Kit-8 (CCK-8, Dojindo, Kumamoto, Japan). After adding 10 µl of CCK-8 reagent per 100 µl of culture medium and culturing for 1 hour in the incubator controlled at a temperature of 37°C in the presence of 5% CO₂, the optical density (O.D.) value was measured by absorbance at 450 nm using a SpectraMax iD3 microplate reader (Molecular Devices, San Jose, CA, USA), and the derived value was converted into a percentage.

### 3. Effect of therapeutic protein CTP alpha on cell viability of each cell line

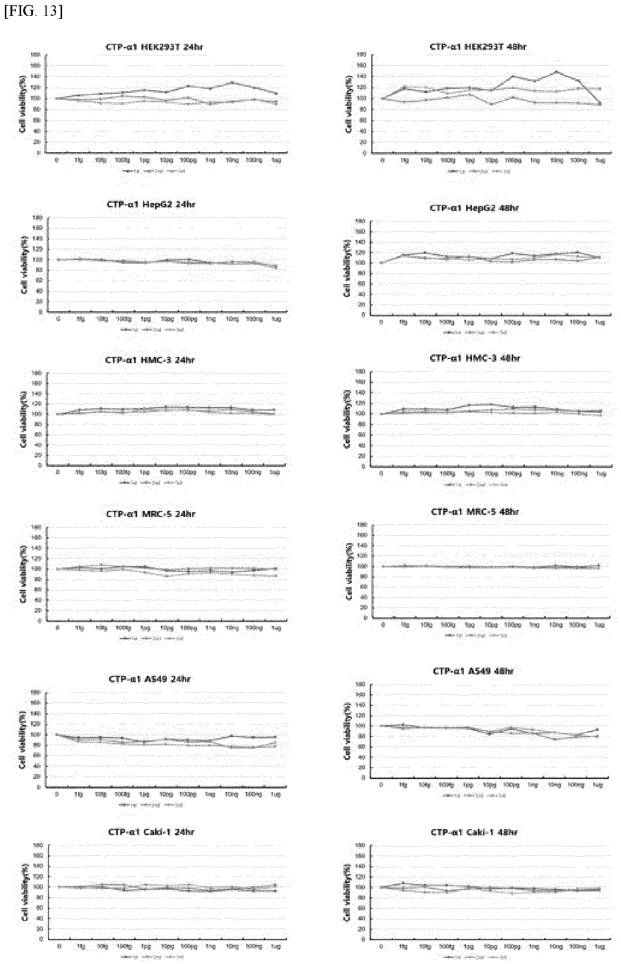

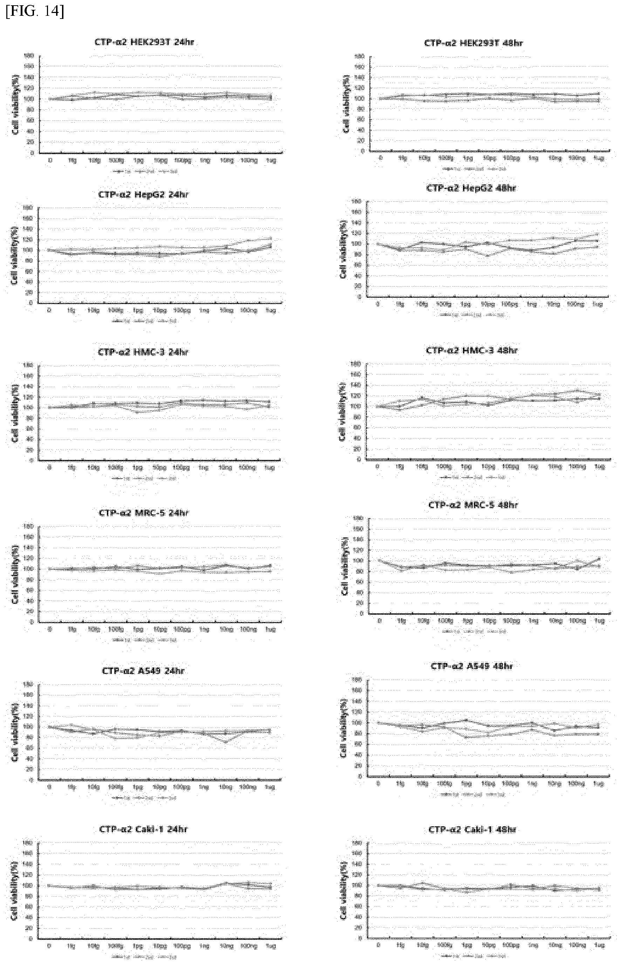

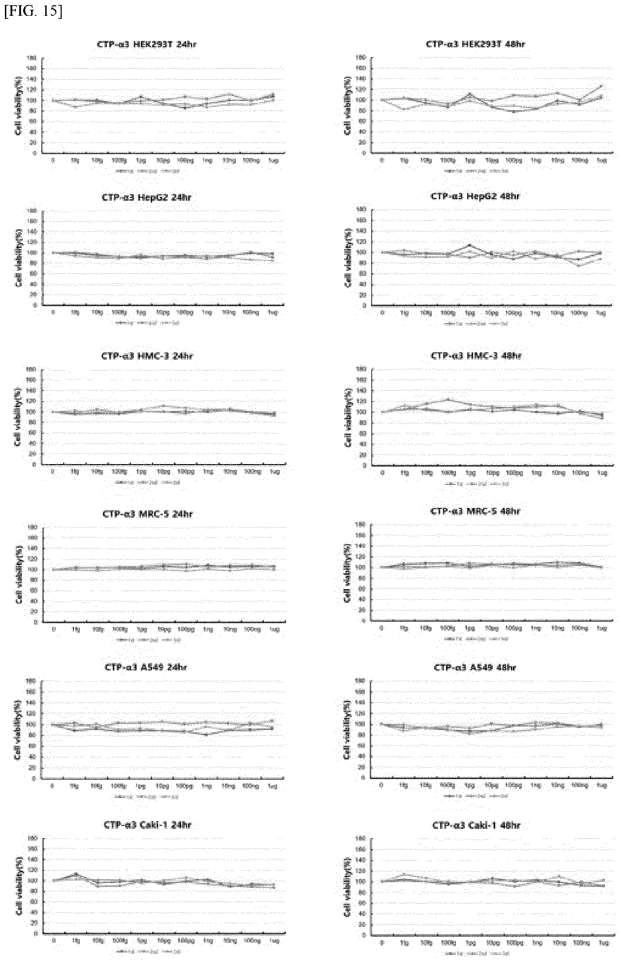

In the present disclosure, a CCK-8 assay was performed to check the toxicity of CTP alpha in 6 cell lines including HEK293T, HepG2, HMC-3, MRC-5, A549, and Caki-1 on the cell survival of each cell line. As a result, after treatment with CTP alpha for 24 hours, when the cell viability of each cell line was checked, no significant change was observed in all 6 cell lines compared to the control group not treated with CTP alpha. As a result, after treatment with CTP alpha for 48 hours, when the cell viability of each cell line was checked, no significant change was observed in all 6 cell lines (FIGS. 13 to 15). Overall, the cytotoxicity analysis results proposed applicability of CTP alpha for preventing and treating coronavirus by identifying the nontoxicity and safety of the CTP alpha.

### <Example 7> 3D structural analysis of a protein of coronavirus infectious disease COVID-19 therapeutic protein CTP beta

### 1. Preparation of a 3D structure of a protein

A binding structure of hACE2-RBD registered in Protein Data Bank (PDB, https://www.rcsb.org/) (PDB ID: 6M0J) was used. The structure of a P6 moiety was modeled using MODELLER based on the binding structure of hACE2-RBD using the sequence of 22-44-G-351-357 of hACE2.

### 2. Molecular dynamic simulation

For the modeled hACE2-RBD complex, P6-RBD complex, CTP beta monomer-RBD complex, CTP beta dimer-RBD complex, P6, CTP beta monomer, and CTP beta dimer, molecular dynamics simulations to which the ff14SB force field was applied were performed using the AMBER18 package. The simulation was performed with the pmemd.cuda program included in AMBER18, and an octahedron TIP3P water box with a length of 15 Å added to the outer portion of the prepared protein was applied for the initial structure for the simulation. A periodic boundary condition was applied based on the water box, and Na+ and Cl- ions were added to neutralize the net charge. The particle-mesh Ewald (PME) method was applied for electromagnetic force of long distance based on a distance of 9 Å. The SHAKE algorithm to fix the distance of a covalent bond of hydrogen molecules was used, and the simulation was performed with a time step of 2 fs/step. First, a 5000-step minimization simulation was performed by applying 0.5 kcal/mol of a position restraint to the backbone structure of the protein, and a heating simulation was performed for 25 ps under NVT ensemble conditions from 10K to 300K by applying 0.1 kcal/mol of a position restraint. Equilibrium simulations were performed under NPT ensemble conditions at a temperature of 300K for 1 ns. The production run was performed under NPT ensemble conditions at a temperature of 300K and a pressure of 1 bar, and the simulation was performed for 500 ns for a complex with RBD and 1 µs for CTP beta alone. Simulations were performed with 5 independent trajectories for each case.

The binding energy of RBD and CTP beta were calculated by the MMGBSA algorithm using simulation snapshots within 300 to 500 ns, and entropy was calculated via normal mode analysis. The MMPBSA.py program in the AMBER18 package was used to calculate the binding free energy.

### 3. Structure of CTP beta

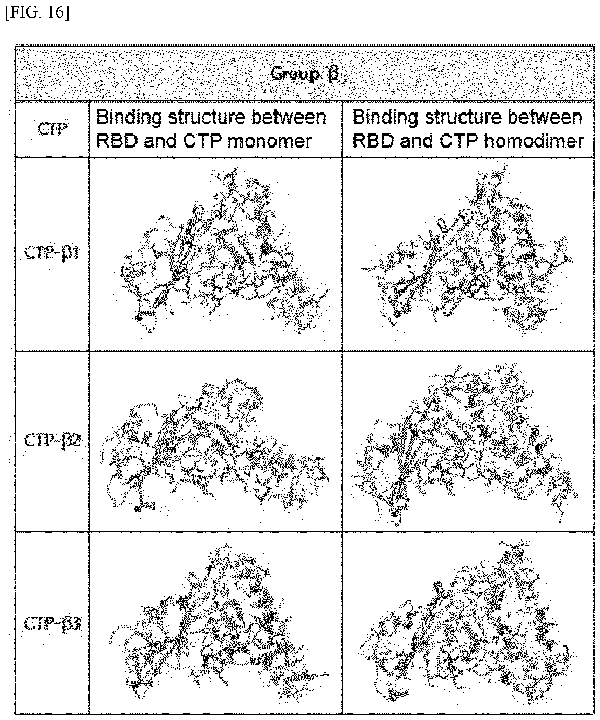

The modeled CTP beta group (CTP-β1, CTP-β2, CTP-β3) has a structure of HTH primarily improved type. When the structural ensemble of the modeled structure of the monomer and dimer forms in a solution was analyzed through molecular dynamics simulation, the structural patterns shown in FIG. 16 were observed.

CTP-β1 is a therapeutic protein with K2 subjected to interaction with D420 of RBD as well as E4 subjected to interaction with K458, both positioned at the N-terminal portion. It is a therapeutic protein with an unstable secondary structure of N-terminal.

CTP-β2 is a therapeutic protein with E2 and E4 subjected to interaction with K458 of RBD positioned at the N-terminal portion. It is a therapeutic protein with an unstable secondary structure of N-terminal.

CTP-β3 is a therapeutic protein with K2 subjected to interaction with D420 of RBD as well as E4 subjected to interaction with K458, both positioned at the N-terminal portion. It is a therapeutic protein with improved stability in the secondary structure of CTP-β2.

### 4. Prediction of binding free energy of CTP beta-RBD

Binding energy and binding free energy were calculated to determine how well CTP beta binds to RBD (Table 9). Using molecular dynamics simulation, structural ensembles in which hACE2, P6, and CTP betas bind to RBD, respectively, were collected, and the binding energy (ΔE) and binding free energy (ΔG) from the structural ensemble were analyzed using the MMGBSA algorithm.

It was found that CTPs have negative binding free energy and are stable when bound to RBD, bind with stronger energy (ΔE) than hACE2 and P6 in simulations due to interaction with a new epitope, and also bind with similar or stronger free energy (ΔG).

**TABLE 9**

| Group | CTP | RBD + CTP monomer | | RBD + CTP dimer | |
|---|---|---|---|---|---|
| | | ΔE | ΔG | ΔE | ΔG |
| - | hACE2 | -74.0327 | - | - | - |
| - | P6 | -63.9558 | -11.0060 | - | - |
| CTP β | CTP-β1 | -59.4622 | -12.0184 | -69.4741 | -12.6848 |
| | CTP-β2 | -80.5331 | -21.8774 | -67.0206 | -7.9079 |
| | CTP-β3 | -70.3415 | -9.0865 | -96.2066 | -23.6538 |

### 5. In-silico immunogenicity analysis of CTP beta

In-silico immunogenicity was analyzed using the globally well-established NetMHC-4.0 server (http://www.cbs.dtu.dk/services/NetMHC/). For the amino acid sequence of CTP beta, prediction was made on the binding of peptide-MHC class I for 81 MHC alleles of each different individual (human) and 41 alleles of animals (monkey, cattle, pig, and mouse) in the NetMHC4.0 server. By searching in peptide units of 13-14 amino acids in length, the total number of searched peptides and the number of peptides expected to have strong binding (SB) and weak binding (WB) thereamong were counted, and the ratio was calculated by (SB+WB)/Total to verify immunogenicity.

It indicates that the lower the ratio to the total (SB+WB)/Total is, the less the antigen-antibody reaction occurs with other proteins when the neutralizing therapeutic protein of the present disclosure is injected into the body (Tables 10 to 15).

**TABLE 10**

| Human | | | | | | |
|---|---|---|---|---|---|---|
| Group | CTP | scan length | Total | SB | WB | (SB+WB)/Total |
| CTP β | CTP-β1 | 13mer | 3402 | 6 | 20 | 0.008 |
| | | 14mer | 3321 | 9 | 23 | 0.010 |
| | CTP-β2 | 13mer | 3402 | 7 | 22 | 0.009 |
| | | 14mer | 3321 | 10 | 26 | 0.011 |
| | CTP-β3 | 13mer | 3402 | 7 | 25 | 0.009 |
| | | 14mer | 3321 | 11 | 30 | 0.012 |

**TABLE 11**

| Chimpanzee | | | | | | |
|---|---|---|---|---|---|---|
| Group | CTP | scan length | Total | SB | WB | (SB+WB)/Total |
| CTP β | CTP-β1 | 13mer | 336 | 0 | 2 | 0.006 |
| | | 14mer | 328 | 0 | 4 | 0.012 |
| | CTP-β2 | 13mer | 336 | 0 | 2 | 0.006 |
| | | 14mer | 328 | 0 | 3 | 0.009 |
| | CTP-β3 | 13mer | 336 | 0 | 2 | 0.006 |
| | | 14mer | 328 | 0 | 3 | 0.009 |

**TABLE 12**

| Rhesus macaque | | | | | | |
|---|---|---|---|---|---|---|
| Group | CTP | scan length | Total | SB | WB | (SB+WB)/Total |
| CTP β | CTP-β1 | 13mer | 756 | 4 | 19 | 0.030 |
| | | 14mer | 738 | 6 | 27 | 0.045 |
| | CTP-β2 | 13mer | 756 | 4 | 19 | 0.030 |
| | | 14mer | 738 | 6 | 27 | 0.045 |
| | CTP-β3 | 13mer | 756 | 4 | 21 | 0.033 |
| | | 14mer | 738 | 6 | 28 | 0.046 |

**TABLE 13**

| Mouse | | | | | | |
|---|---|---|---|---|---|---|
| Group | CTP | scan length | Total | SB | WB | (SB+WB)/Total |
| CTP β | CTP-β1 | 13mer | 252 | 3 | 10 | 0.052 |
| | | 14mer | 246 | 2 | 14 | 0.065 |
| | CTP-β2 | 13mer | 252 | 3 | 10 | 0.052 |
| | | 14mer | 246 | 2 | 14 | 0.065 |
| | CTP-β3 | 13mer | 252 | 1 | 13 | 0.056 |
| | | 14mer | 246 | 2 | 16 | 0.073 |

**TABLE 14**

| BoLA | | | | | | |
|---|---|---|---|---|---|---|
| Group | CTP | scan length | Total | SB | WB | (SB+WB)/Total |
| CTP β | CTP-β1 | 13mer | 252 | 0 | 0 | 0.000 |
| | | 14mer | 246 | 0 | 0 | 0.000 |
| | CTP-β2 | 13mer | 252 | 0 | 0 | 0.000 |
| | | 14mer | 246 | 0 | 0 | 0.000 |
| | CTP-β3 | 13mer | 252 | 0 | 0 | 0.000 |
| | | 14mer | 246 | 0 | 0 | 0.000 |

**TABLE 15**

| Pig | | | | | | |
|---|---|---|---|---|---|---|
| Group | CTP | scan length | Total | SB | WB | (SB+WB)/Total |
| CTP β | CTP-β1 | 13mer | 126 | 0 | 0 | 0.000 |
| | | 14mer | 123 | 0 | 1 | 0.008 |
| | CTP-β2 | 13mer | 126 | 0 | 0 | 0.000 |
| | | 14mer | 123 | 0 | 1 | 0.008 |
| | CTP-β3 | 13mer | 126 | 0 | 0 | 0.000 |
| | | 14mer | 123 | 0 | 1 | 0.008 |

### <Example 8> Cloning of coronavirus infectious disease COVID-19 therapeutic protein CTP beta

### 1. Strain and medium

All chemicals used for gene cloning were of analytical grade. Completed clones were transformed using a DH5α E. coli strain for proliferation and screened with LB medium in which ampicillin (LPS, 100 µg/ml) was added. Cell culture was performed at 37°C with stirring involved.

### 2. Construction of plasmids

After converting the amino acid sequence of the protein designed in silico into nucleotides using the Sequence Manipulation Suite (https://www.bioinformatics.org/sms2/rev_trans.html) tool, DNA sequences were determined by comparing with the sequence present in hACE2. The synthesized DNA fragment (Bioneer, gene synthesis) was amplified by PCR using a forward primer 5'-GGAGATATACATATGAAAAGTGAACTTGCTTCTGTGAATTAT(CTP-β1), 5'-GGAGATATACATATGGAAAGTGAACTTGCTTCTGTGAA(CTP-β2), and 5'-GGAGATATACATATGGAAAGTGAACTTGCTTCTAATGTG(CTP-β3) as well as a common reverse primer 5'-GTGGTGCTCGAGCCTGAAGTCGCCCTTCC, and then inserted into a pET-21a vector treated with restriction enzymes Nde I and Xho I by ligation independent cloning using EZ-Fusion^{™} HT Cloning Kit (Engnomics).

### 3. Experiment result

The therapeutic protein CTP beta of the present disclosure is a therapeutic protein of a new amino acid sequence that neutralizes or inhibits the S1-RBD protein from binding to the hACE2 protein, wherein the amino acid sequence of the therapeutic protein CTP beta designed in silico is as shown in Table 16.

**TABLE 16**

| Group β | |
|---|---|
| CTP name | Sequence |
| CTP-β1 | MKSELASVNYNTNITKENVQANGEEQAKTFLDKFNHEAEDLFY QSSGLGKGDFR (SEQ ID NO: 4) |
| CTP-β2 | MESELASVNYNTNITKENVQANGEEQAKTFLDKFNHEAEDLFYQ SSGLGKGDFR (SEQ ID NO: 5) |
| CTP-β3 | MESELASNVYNTNITKENVQNMNEEQAKTFLDKFNHEAEDLFY QSSGLGKGDFR (SEQ ID NO: 6) |

### <Example 9> Expression and purification of coronavirus infectious disease COVID-19 therapeutic protein CTP beta

### 1. Protein expression and purification materials

All chemicals used for protein expression and purification were of analytical grade. Ampicillin, 1-thio-β-d-galactopyranoside (IPTG), sodium phosphate-monobasic, sodium phosphate-dibasic, and TRIS used for protein expression and purification were purchased from LPS (Daejeon, Korea), Coomassie brilliant blue R-250, 2-mercaptoethanol, chloramphenicol, and imidazole were purchased from Biosesang (Seongnam, Korea), sodium chloride was purchased from Duksan (Daejeon, Korea), and all columns were purchased from GE Healthcare (Piscataway, NJ). Phenylmethanesulfonyl fluoride (PMSF), trizma hydrochloride, and Amicon Ultra-15 Centrifugal Filter Units used herein were purchased from Millipore (Billerica, MA).

### 2. Protein expression and purification instruments

Shake incubator (NBS/Innova 42R) and high-performance high-capacity centrifuge (Beckman Coulter. Inc./AVANTI JXN-26) were used for protein expression, and protein high-speed separation system (GE Healthcare/AKTA Pure M and Start System), protein high-speed separation system (GE HealthcareIFPLC Accessory System), ultrasonicator (Q700-Sonicator), and refrigerated centrifuge (Epoendorf-5810R) were used to isolate and purify proteins.

### 3. Expression and culture of therapeutic protein CTP beta

The gene cloned in the pET-21a vector was used for transformation for expression using a BL21 (DE3) RIL E. coli strain, and cell culture was performed at 37°C using LB medium in which ampicillin (100 mg/ml) and chloramphenicol (50 mg/ml) were added. After culture to meet OD₆₀₀ value of 0.6-0.8 (OD₆₀₀=0.6~0.8), 1M IPTG was added to a final concentration of 0.5 mM, followed by expression at 37°C for 4 hours. A high-performance high-capacity centrifuge (Beckman Coulter. Inc./AVANTI JXN-26) set at 7000 RPM, 4°C was used to harvest the cells for 20 minutes to be used immediately or stored at -80°C.

### 4. Isolation and purification of therapeutic protein CTP beta

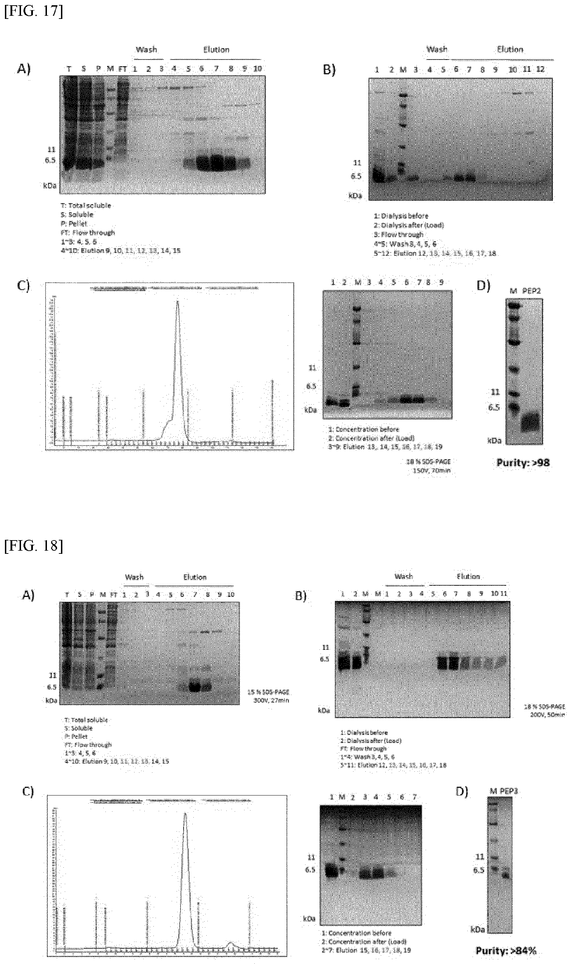

After dissolving in 10 ml of lysis buffer (pH 8.0, 50 mM Tris-HCl, 500 mM NaCl, 1 mM PMSF, 0.25% Tween-20 (v/v)) based on 1 g of cells, the cells were disrupted using a sonicator (Q700-Sonicator). In order to isolate the solution and the pellet, a high-speed centrifuge set at 18000 RPM and 4°C was used for 30 minutes. A 0.45 µm syringe filter was used for removal of impurities in the aqueous solution. The solution from which impurities were removed was poured through a HisTrap HP 5ml (GE Healthcare) column stabilized with wash buffer (pH 8.0, 50 mM Tris-HCl, 500 mM NaCl), and then elution buffer (pH 8.0, 50 mM Tris-HCl, 500 mM NaCl, 500 mM imidazole) was set to 0 to 100% gradient elution. As a result, it was found that the therapeutic protein CTP beta was isolated with imidazole at a concentration of 100 to 300 mM. The isolated therapeutic protein CTP beta was subjected to buffer exchange (pH 8.0, 20 mM Tris-HCl) in a cold room for 15 hours. After pouring the NaCl-removed CTP beta solution through the HiTrap Q HP 5ml (GE Healthcare) column stabilized with binding buffer (pH 8.0, 20 mM Tris-HCl), elution buffer (pH 8.0, 20 mM Tris-HCl, 1.0 M NaCl) was set from 0 to 100% gradient elution. As a result, it was found that the therapeutic protein CTP beta was isolated with NaCl at a concentration of 120 to 250 mM. Finally, therapeutic protein CTP beta having a dimer structure was purely isolated and purified using a HiLoad Superdex 75 16/600 column stabilized with pH 7.4, 10 mM phosphate buffer and 150 mM NaCl. Quantification was performed at A₂₈₀ₙₘ using NanoDrop, and, as a result of calculating the purity using the ImageJ program, high purity (>84%) therapeutic protein CTP beta protein was obtained (FIGS. 17 to 19).

### <Example 10> Mass spectrometry of coronavirus infectious disease COVID-19 therapeutic protein CTP beta

### 1. Mass spectrometry analysis

Mass spectrometry was used to identify the total mass and information on the amino acid sequence of the protein. Mass spectrometry of total proteins was performed using a high-performance matrix laser desorption ionization mass spectrometer (Bruker, UltrafleXtreme MALDI TOF/TOF). Hybrid triple quadrupole linear ion trap mass spectrometer (Sciex, QTRAP) and quadrupole orbitrap mass spectrometer (Thermo scientific, Q Exactive PLUS) were used for amino acid sequence analysis for the protein and micro liquid chromatography (Sciex, M5 MicroLC) was used for QTRAP, and nano-micro liquid chromatography (Waters, ACQUITY UPLC M-Class system) was used for Q Exactive PLUS.

### 2. Sample preparation for MALDI TOF and analysis of intact proteins using MALDI TOF

For total mass spectrometry of ACE2, RBD, and CTP beta proteins, 10 µg of protein obtained by removing low-molecular compounds using Microcon centrifugal filter (Merck, MRCPRT010) and sinapinic acid (Bruker, 8201345) matrix were mixed in a 1:1 ratio, and the mixture was then placed on a target plate to dry and subjected to the flexcontrol program of the high-performance matrix laser desorption ionization mass spectrometer. The mass value was normalized using the Starter kit for MALDI-TOF MS (Bruker, 8208241), and the spectrum was obtained with an average of 1000 laser shots based on the method provided by flexcontrol. The result values were derived from each spectral data using the flexanalysis program.

### 3. Sample preparation for LC-MS/MS analysis

For amino acid sequence analysis of a single protein, 20 µg of RBD, hACE2, and CTP beta proteins were mixed with 100 mM triethylammonium bicarbonate (Sigma, T7408-100ML) and 2% sodium dodecyl sulfate (Duksan, 6645) to a specific concentration. After adding 10 mM dithiothreitol (BIO-RAD, 161-0611) and treating at 56°C for 30 minutes, 20 mM iodoaetamide (Sigma, T1503) was added and shielded from light, followed by treatment at room temperature for 30 minutes. Then, using S-trap mini columns (PROTIFI, C02-mini-80), low molecular weight compounds were removed, and 2 µg of proteases including trypsin (Promega, V5111) and Glu-C (Promega, V1651) were added, followed by enzymatic degradation at 37°C for 16 hours. After termination of the reaction, isolation was performed with a centrifuge, drying was followed under vacuum, and 0.1% trifluoroacetic acid was added to prepare a sample. In addition, deglycosylation experiments were added for RBD and ACE2 proteins. For deglycosylation experiment, 100 mM triethylammonium bicarbonate and 2 µg of PNGase F (Promega, V4831) were added to the protease-treated sample, followed by enzymatic degradation at 37°C for 4 hours. After termination of the reaction, the proteins were isolated by centrifuge using S-trap mini columns. Vacuum drying was followed, and then 0.1% trifluoroacetic acid was added to prepare a sample. For the RBD and hACE2 proteins, a quadrupole orbitrap mass spectrometer was used, and experiments proceeded via nano-micro liquid chromatography. For the CTP beta protein, a hybrid triple quadrupole linear ion trap mass spectrometer was used, and experiments proceeded using micro-liquid chromatography.

### 4. LC-MS/MS Analysis: ACQUITY UPLC M-Class system - Q Exactive PLUS

All solvents used for liquid chromatography were of HPLC grade. Water (Duksan, 7732-18-5) and acetonitrile (Honeywell, AH015-4) were used as solvents, and dimethyl sulfoxide (Sigma, 472301-1L), trifluoroacetic acid (Thermo scientific, 28904), and formic acid (Thermo scientific, 28905) were used as a medium in the experimental solution. Nano-micro liquid chromatography was composed of solvent A (0.1% formic acid, 5% dimethyl sulfoxide in water) and solvent B (0.1% formic acid, 5% dimethyl sulfoxide in 80% acetonitrile). Acclaim^{™} PepMap^{™} 100 C18 LC Column (Thermo scientific, 164197) was used as a trapping column, and EASY-Spray^{™} HPLC Columns (Thermo scientific, ES803A) as an analytical column. Liquid chromatography was performed for 5 minutes in the presence of 95% solvent A and 5% solvent B at a flow rate of 8 µl/min in the trapping step after injecting 0.5 µg of the sample. In the analytical step, the flow rate was 250 nl/min. After 5 minutes under the conditions of 95% solvent A and 5% solvent B, the samples were analyzed by gradually increasing the rate of solvent B from 5% to 40% for 140 minutes, the column was activated for 15 minutes after increasing to 95% for 5 minutes again, and re-equilibration was followed by decreasing the rate of solvent B to 5% for 5 minutes and maintaining the state for 40 minutes. The mass values of quadrupole orbitrap mass spectrometer were calibrated by Pierce^{™} LTQ Velos ESI Positive Ion Calibration Solution (Thermo scientific, 88323). The analysis was conducted via full-MS and data dependent MS/MS methods. The calculated data was obtained using a proteome discoverer (Thermo scientific).

### 5. LC-MS/MS Analysis: M5 MicroLC - QTRAP

The column used for the analysis was Kinetex 2.6u XB-C18 100A (Phenomenex, 00B-4496-AC). Micro liquid chromatography was composed of solvent A (0.1% formic acid in water) and solvent B (0.1% formic acid in acetonitrile). The liquid chromatography was performed at a flow rate of 20 µl/min after injecting 1.5 µg of the sample. After 5 minutes under the conditions of 95% solvent A and 5% solvent B, the samples were analyzed by gradually increasing the rate of solvent B from 5% to 40% for 45 minutes, the column was activated for 4 minutes after increasing to 95% for 5 minutes again, and re-equilibration was followed by decreasing the rate of solvent B to 5% for 3 minutes and maintaining the state for 3 minutes. The mass values of hybrid triple quadrupole linear ion trap mass spectrometer were calibrated by standard chemical kit (Sciex, 4406127). The analysis was conducted using Q3 MS and enhanced product ion method using information dependent acquisition. For the calculated data, the wiff file was converted to the mgf file using convert MS (proteowizard.sourceforge.net), and the results were obtained using searchGUI and peptide-shaker (compomics.github.io).

### 6. Results of intact protein mass spectrometry

The mass of the intact protein was measured using MALDI-TOF. The mass of RBD was 30 kDa. In addition to 30kDa, the peak was a dimer, appeared along with a 60kDa peak, a monomer with a charge (z) of 2 (m/z=15kDa), and a trimer with a charge (z) of 2 (m/z=45kDa). Considering that the mass of RBD calculated from the protein sequence is 25.9 kDa, it may be suggested that glycosylation caused the change in mass of 4.1 kDa. Similarly, the mass of hACE2 was measured to be about 102 kDa, and a dimer and a peak with a charge of 2 also appeared together. Considering that the mass of hACE2 calculated from the protein sequence is 84.4 kDa, it is suggested that the mass change of about 17.5 kDa was caused by glycosylation.

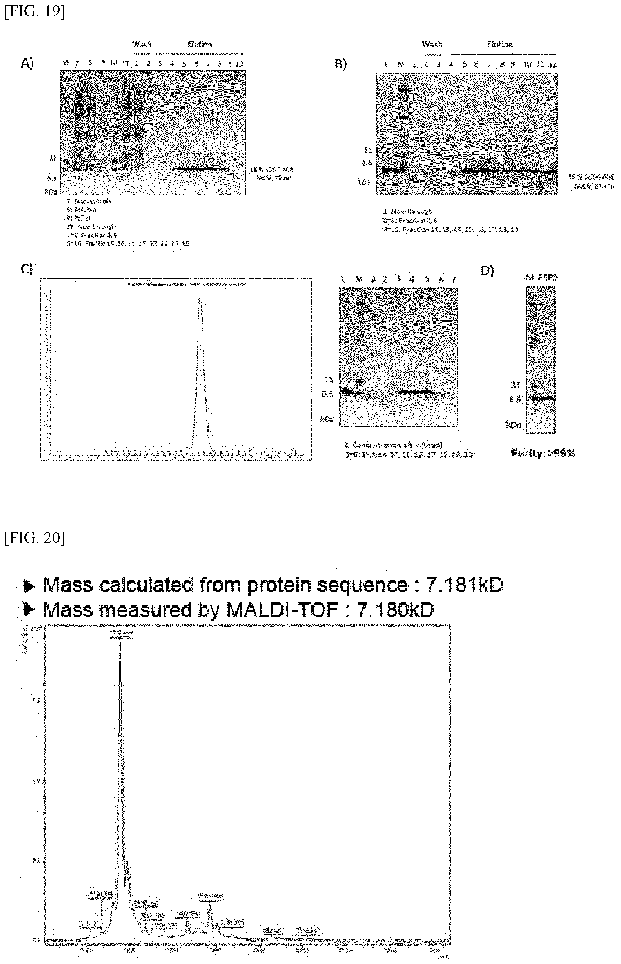

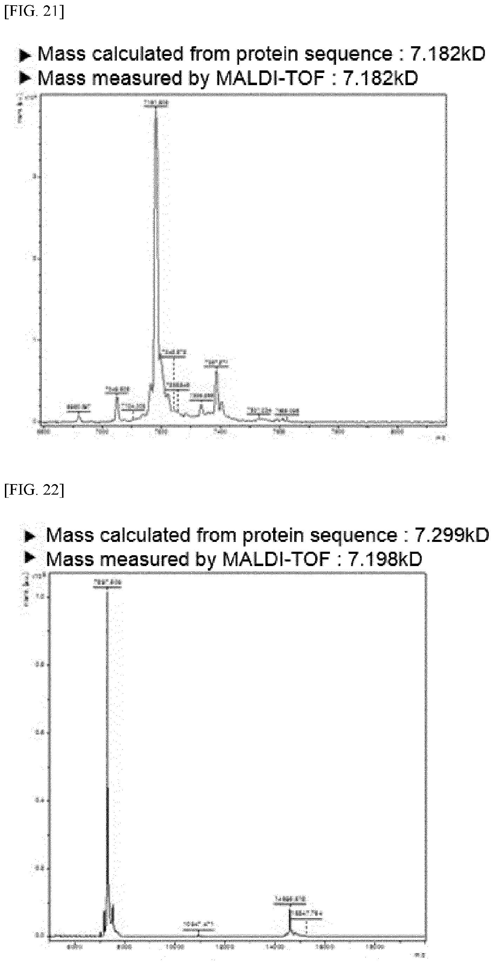

CTP-β1 was measured to be 7.180kD, almost equal to the mass calculated from the sequence (FIG. 20), CTP-β2 was measured to be 7.182kD, almost equal to the mass calculated from the sequence (FIG. 21), and CTP-β3 was measured to be 7.298kD, almost equal to the mass calculated from the sequence (Fig. 22).

### 7. Identification of protein sequences

LC-MS/MS analysis was performed to identify the sequences of RBD, hACE2, and CTP beta. Each protein was subjected to protein analysis using proteases such as trypsin and Glu-C, wherein, for RBD and hACE2, N-glycosylated Asn was found by searching for amino acid residues in which Asn was changed to Asp by enzymatic activity of PNGase F.

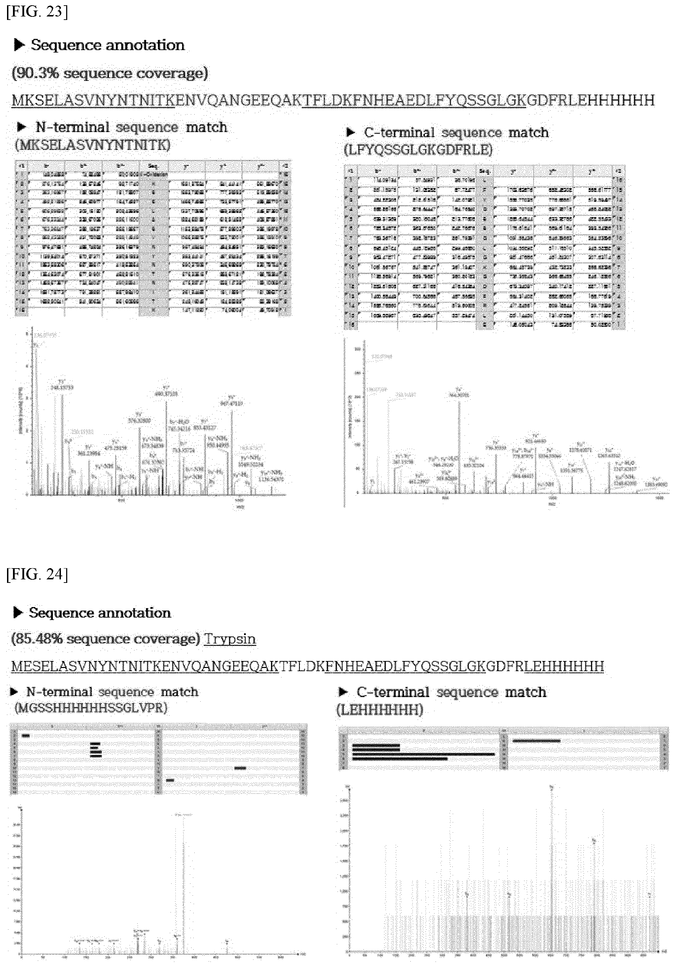

The results of MS/MS analysis of CTP beta with N, C-terminus identified are shown in FIGS. 23 to 25. Sequence analysis of the N, C-terminus is one of the means for identifying the state of the protein, and if the terminus of the protein is not identified, it may be considered as being degraded due to a problem in the preservation state of the protein. In the case of RBD and hACE2, the terminus of the proteins tends not to be identified (ID), so unless inaccuracy in the sequence or the effect of PTM matters, they are easily degraded, so care must be taken for preservation.

### <Example 11> Secondary structural stability and function/efficacy verification of coronavirus infectious disease COVID-19 therapeutic protein CTP beta

### 1. Circular dichroism (CD) experiment

Far UV circular dichroism was measured for RBD (PBS buffer), hACE2 (PBS buffer), and CTP beta. A JASCO-1500 machine was used with the protein concentration of 0.2 mg/ml and a quartz cell of 0.1 cm at a temperature of 20°C. Measurements were performed at a measurement speed of 20 nm/min, a bandwidth of 5 nm, and a digital integration time (D.I.T) of 4 seconds at a far UV wavelength of 190-250 nm. A graph was drawn by subtracting the data obtained by 5 times measurement for the protein by the data obtained by 5 times measurement for the buffer.

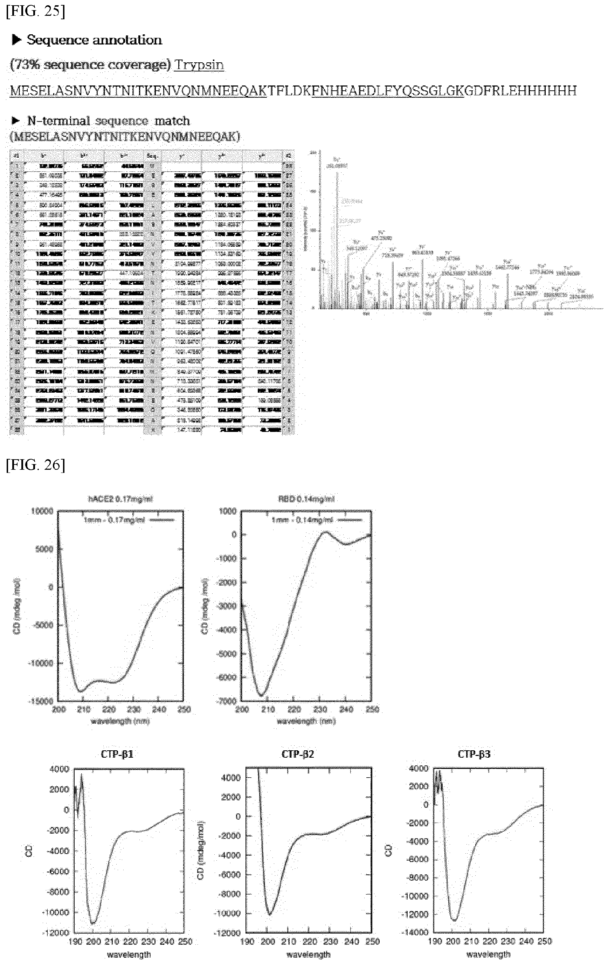

As a result, it was found that hACE2 is a protein having α-helix as a secondary structure, and RBD is a protein having a beta sheet as a secondary structure. It was found that CTP beta has both a helical structure and a coil structure (FIG. 26).

### 2. Microscale thermophoresis (MST) experiment

Fluorescence was attached to 100 µL of 8 µM RBD (PBS buffer with 0.05% Tween-20 added) using Monolith Protein Labeling Kit RED-NHS 2nd Generation. Ligand (hACE2 (PBS buffer) and CTP beta were prepared by 10 µL each with 16 concentrations from 16 µM to 0.488 nM using a 1:1 serial dilution. A mixed solution of 15 nM RBD and 8 µM-0.244 nM ligand was prepared by diluting the fluorescence-attached RBD to 30 nM and adding 10 µL of the RBD to each ligand of 16 concentrations. MST was measured 5 times for each ligand using a Monolith NT.115 machine with 60% excitation power and medium MST power. Kd (dissociation constant) value was calculated using Kdmodel of MO affinity analysis program provided by Monolith company. Experimental results having the median Kd value derived from 5 experiments are shown in a graph.

As a result, a change in concentration-dependent fluorescence intensity was observed, indicating that both hACE2 and CTP beta bind to RBD. The Kd values representing the binding strength were 82 nM for CTP-β1, 42.9 nM for CTP-β2, 56.4 nM for CTP-β3, and 42.2 nM for hACE2 (FIG. 12). The Kd values of RBD and hACE2 are similar to that of 34.6 nM measured by using BLI (biolayer interferometry) and SPR (Surface Plasmon Resonance) in the Science paper (Wrapp et al, Science 367, 1260-1263 (2020)).

### <Example 12> Toxicity test for coronavirus infectious disease COVID-19 therapeutic protein CTP beta

### 1. Cell culture for toxicity analysis of therapeutic protein CTP beta

The cell line used in the present disclosure was obtained from the Korea Cell Line Bank (KCLB, Seoul, Korea). Used as a culture medium for the human embryonic kidney cell line HEK293T, the human liver cancer cell line HepG2, and the human lung cancer cell line A549 was Dulbecco's modified eagle medium (DMEM, Hyclone, USA) supplemented with 10% fetal bovine serum (FBS, Hyclone, USA) and 1% antibiotic (penicillin-streptomysin; P/S, Gibco, USA), and human microglia HMC-3 was cultured in Minimum Essential Media (MEM, Gibco, USA) supplemented with 10% fetal bovine serum and 1% antibiotic (penicillin-streptomycin). Used as a culture medium for human normal lung cell line MRC-5 was Minimum Essential Media (MEM) supplemented with 10% fetal bovine serum, 1% antibiotic (penicillin-streptomycin) and 25 µM hydroxyethyl piperazine ethane sulfonic acid (HEPES, Hyclone, USA). For the human kidney cancer cell line Caki-1, McCoy's 5A medium (Gibco, USA) supplemented with 10% fetal bovine serum and 1% antibiotics (penicillin-streptomycin) was used as a culture medium. All cell lines used were cultured in an incubator controlled to a temperature of 37°C in the presence of 5% CO₂.

### 2. Cytotoxicity analysis for therapeutic protein CTP beta

The HEK293T, HepG2, HMC-3, MRC-5, A549, and Caki-1 cell lines were dispensed into 96-well cell culture plates in the count of 3×10³, 6×10³, 5×10³, 6×10³, 5×10³, and 5×10³ cells/well, respectively, and then stabilized in an incubator controlled at a temperature of 37°C in the presence of 5% CO₂ for 24 hours. Then, a starvation culture medium in which the FBS content was reduced from 10% to 1% in the culture medium composition for each cell line was prepared and replaced with a starvation culture medium, followed by starvation for 16 hours. Thereafter, 1 fg, 10 fg, 100 fg, 1 pg, 10 pg, 100 pg, 1 ng, 10 ng, 100 ng, 1 µg, and 10 µg of CTP beta were added to 100 µl of the culture medium, respectively, and the mixture was treated to the cell line, followed by culture for 24 hours and 48 hours. Viability of the cell lines was measured using Cell Counting Kit-8 (CCK-8, Dojindo, Kumamoto, Japan). After adding 10 µl of CCK-8 reagent per 100 µl of culture medium and culturing for 1 hour in the incubator controlled at a temperature of 37°C in the presence of 5% CO₂, the optical density (O.D.) value was measured by absorbance at 450 nm using a SpectraMax iD3 microplate reader (Molecular Devices, San Jose, CA, USA), and the derived value was converted into a percentage.

### 3. Effect of therapeutic protein CTP beta on cell viability of each cell line

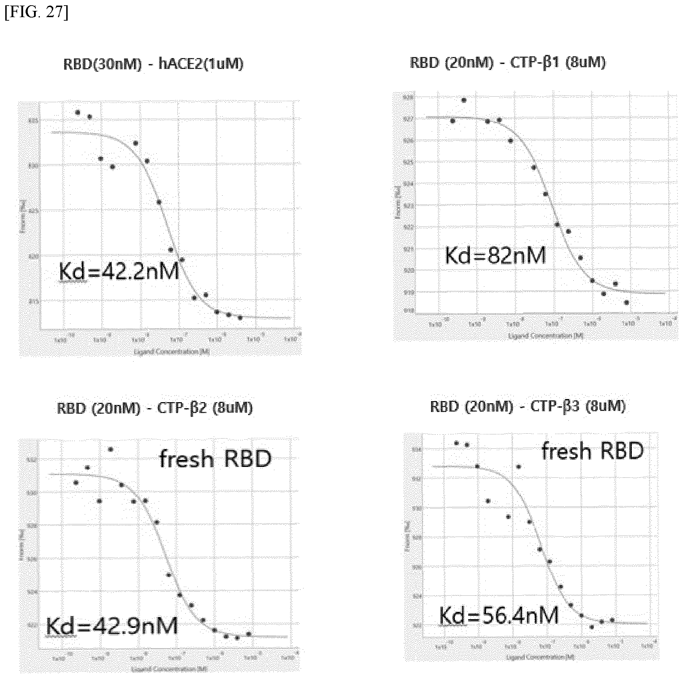

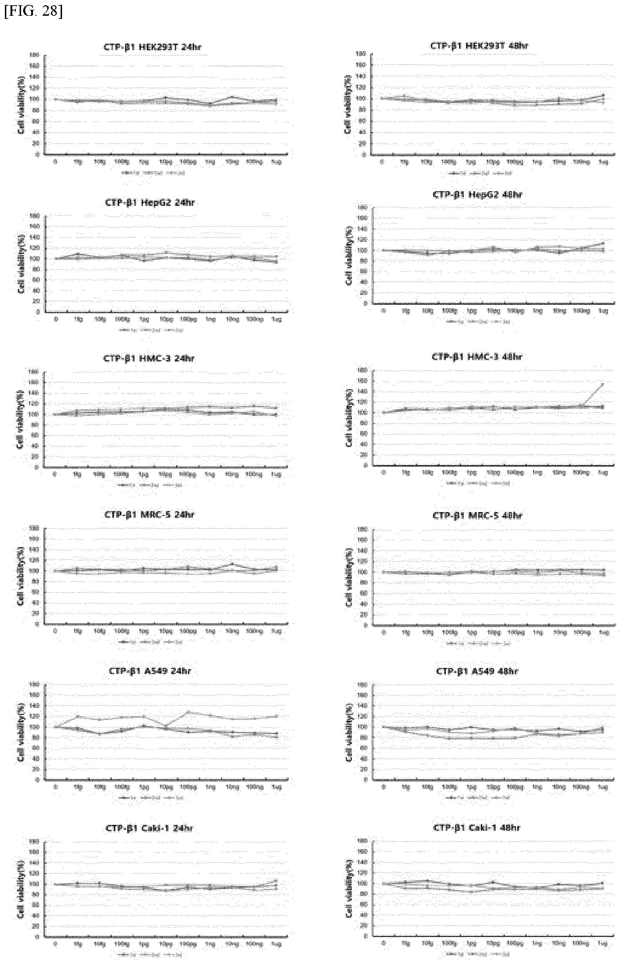

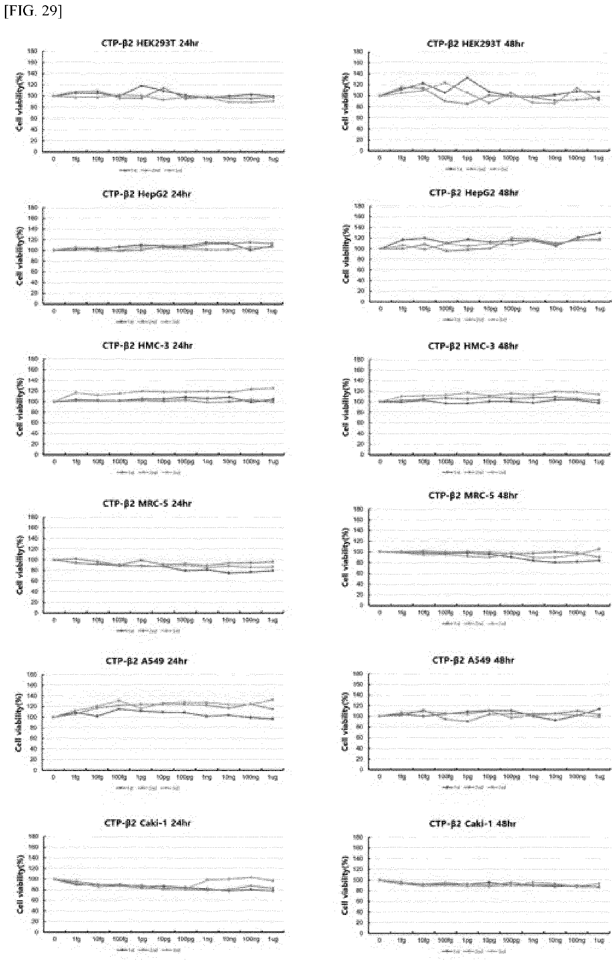

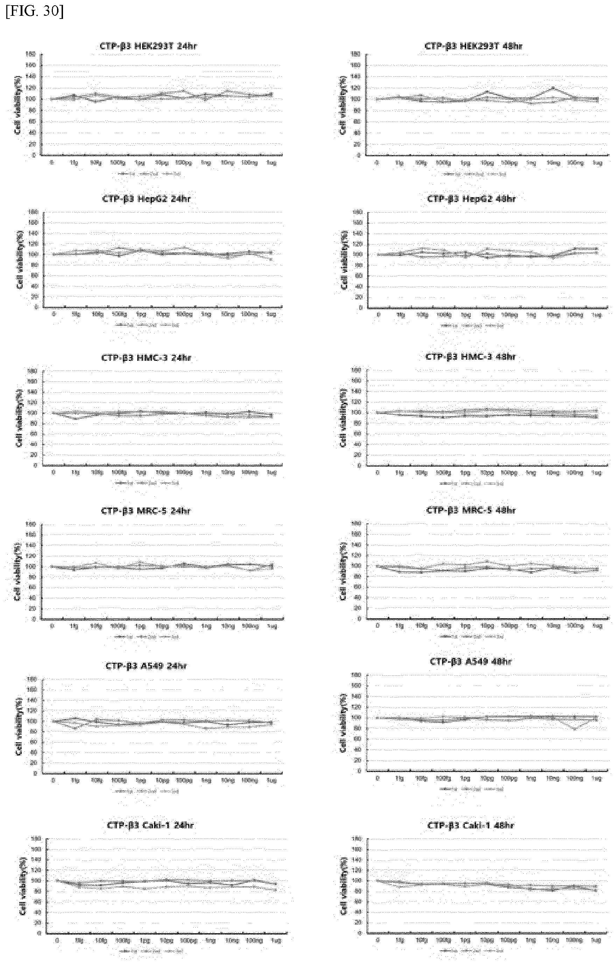

In the present disclosure, a CCK-8 assay was performed to check the toxicity of CTP beta in 6 cell lines including HEK293T, HepG2, HMC-3, MRC-5, A549, and Caki-1 on the cell survival of each cell line. As a result, after treatment with CTP beta for 24 hours, when the cell viability of each cell line was checked, no significant change was observed in all 6 cell lines compared to the control group not treated with CTP beta. In addition, after treatment with CTP beta for 48 hours, when the cell viability of each cell line was checked, no significant change was observed in all 6 cell lines (FIGS. 27 to 30). Overall, the cytotoxicity analysis results proposed applicability of CTP beta for preventing and treating coronavirus by finding the nontoxicity and safety of the CTP beta.

### <Example 13> 3D structural analysis of a protein of coronavirus infectious disease COVID-19 therapeutic protein CTP gamma

### 1. Preparation of a 3D structure of a protein

A binding structure of hACE2-RBD registered in Protein Data Bank (PDB, https://www.rcsb.org/) (PDB ID: 6M0J) was used. The structure of a P6 moiety was modeled using MODELLER based on the binding structure of hACE2-RBD using the sequence of 22-44-G-351-357 of hACE2.

### 2. Molecular dynamic simulation

For the modeled hACE2-RBD complex, P6-RBD complex, CTP gamma monomer-RBD complex, CTP gamma dimer-RBD complex, P6, CTP gamma monomer, and CTP gamma dimer, molecular dynamics simulations to which the ff14SB force field was applied were performed using the AMBER18 package. The simulation was performed with the pmemd.cuda program included in AMBER18, and an octahedron TIP3P water box with a length of 15 Å added to the outer portion of the prepared protein was applied for the initial structure for the simulation. A periodic boundary condition was applied based on the water box, and Na+ and Cl- ions were added to neutralize the net charge. The particle-mesh Ewald (PME) method was applied for electromagnetic force of long distance based on a distance of 9 Å. The SHAKE algorithm to fix the distance of a covalent bond of hydrogen molecules was used, and the simulation was performed with a time step of 2 fs/step. First, a 5000-step minimization simulation was performed by applying 0.5 kcal/mol of a position restraint to the backbone structure of the protein, and a heating simulation was performed for 25 ps under NVT ensemble conditions from 10K to 300K by applying 0.1 kcal/mol of a position restraint. Equilibrium simulations were performed under NPT ensemble conditions at a temperature of 300K for 1 ns. The production run was performed under NPT ensemble conditions at a temperature of 300K and a pressure of 1 bar, and the simulation was performed for 500 ns for a complex with RBD and 1 µs for CTP gamma alone. Simulations were performed with 5 independent trajectories for each case.

The binding energy of RBD and CTP gamma were calculated by the MMGBSA algorithm using simulation snapshots within 300 to 500 ns, and entropy was calculated via normal mode analysis. The MMPBSA.py program in the AMBER18 package was used to calculate the binding free energy.

### 3. Structure of CTP gamma

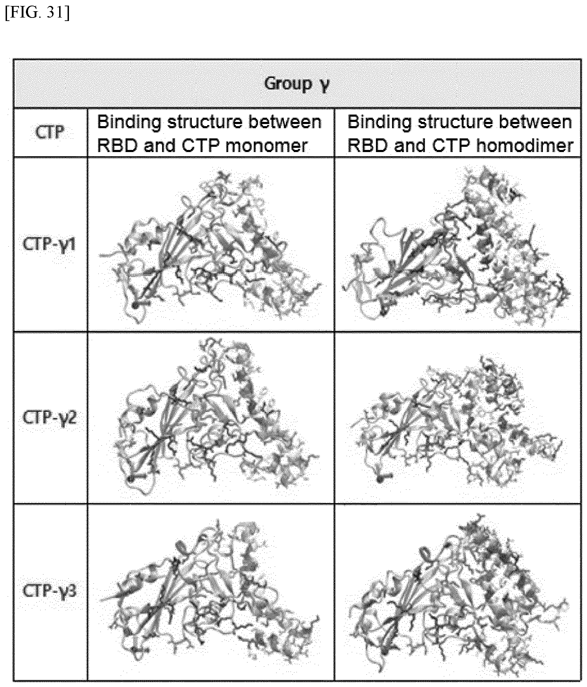

The modeled CTP gamma group (CTP-γ1, CTP-γ2, CTP-γ3) has structures of HTH improved type and 55AA type. When the structural ensemble of the modeled structure of the monomer and dimer forms in a solution was analyzed through molecular dynamics simulation, the structural patterns shown in FIG. 31 were observed.

CTP-γ1 is a therapeutic protein to which S5 was added to increase the length of the therapeutic protein, with K2 subjected to interaction with D420 of RBD as well as D8 subjected to interaction with K458, both positioned at the N-terminal portion. It is a therapeutic protein with improved stability in the secondary structure.

CTP-γ2 is a therapeutic protein to which S5 was added to increase the length of the therapeutic protein, with K2 subjected to interaction with D420 of RBD as well as E3 and D8 subjected to interaction with K458 positioned at the N-terminal portion. It is a therapeutic protein with improved stability in the secondary structure.

CTP-γ3 is a therapeutic protein to which E5 was added to increase the length, with K2 subjected to interaction with D420 of RBD as well as E3 and D8 subjected to interaction with K458 positioned at the N-terminal portion. It is a therapeutic protein with improved stability in the secondary structure.

### 4. Prediction of binding free energy of CTP gamma-RBD

Binding energy and binding free energy were calculated to determine how well CTP gamma binds to RBD (Table 17). Using molecular dynamics simulation, structural ensembles in which hACE2, P6, and CTP gammas bind to RBD, respectively, were collected, and the binding energy (ΔE) and binding free energy (ΔG) from the structural ensemble were analyzed using the MMGBSA algorithm.

It was found that CTPs have negative binding free energy and are stable when bound to RBD, bind with stronger energy (ΔE) than hACE2 and P6 in simulations due to interaction with a new epitope, and also bind with similar or stronger free energy (ΔG).

**TABLE 17**

| Group | CTP | RBD + CTP monomer | | RBD + CTP dimer | |
|---|---|---|---|---|---|
| | | ΔE | ΔG | ΔE | ΔG |
| - | hACE2 | -74.0327 | - | - | - |
| - | P6 | -63.9558 | -11.0060 | - | - |
| CTPγ | CTP-γ1 | -69.3634 | -8.4682 | -104.0715 | -32.8392 |
| | CTP-γ2 | -68.7772 | -6.3356 | -94.4489 | -27.5263 |
| | CTP-γ3 | -63.7947 | -13.5819 | -77.4240 | -12.4105 |

### 5. In-silico immunogenicity analysis of CTP gamma

In-silico immunogenicity was analyzed using the globally well-established NetMHC-4.0 server (http://www.cbs.dtu.dk/services/NetMHC/). For the amino acid sequence of CTP gamma, prediction was made on the binding of peptide-MHC class I for 81 MHC alleles of each different individual (human) and 41 alleles of animals (monkey, cattle, pig, and mouse) in the NetMHC4.0 server. By searching in peptide units of 13-14 amino acids in length, the total number of searched peptides and the number of peptides expected to have strong binding (SB) and weak binding (WB) thereamong were counted, and the ratio was calculated by (SB+WB)/Total to verify immunogenicity.

It indicates that the lower the ratio to the total (SB+WB)/Total is, the less the antigen-antibody reaction occurs with other proteins when the neutralizing therapeutic protein of the present disclosure is injected into the body (Tables 18 to 23).

**TABLE 18**

| Human | | | | | | |
|---|---|---|---|---|---|---|
| Group | CTP | scan length | Total | SB | WB | (SB+WB)/ Total |
| CTP γ | CTP-γ1 | 13mer | 3483 | 8 | 20 | 0.008 |
| | | 14mer | 3402 | 11 | 28 | 0.011 |
| | CTP-γ2 | 13mer | 3483 | 8 | 21 | 0.008 |
| | | 14mer | 3402 | 11 | 30 | 0.012 |
| | CTP-γ3 | 13mer | 3483 | 6 | 28 | 0.010 |
| | | 14mer | 3402 | 10 | 35 | 0.013 |

**TABLE 19**

| Chimpanzee | | | | | | |
|---|---|---|---|---|---|---|
| Group | CTP | scan length | Total | SB | WB | (SB+WB)/ Total |
| CTP γ | CTP-γ1 | 13mer | 344 | 0 | 3 | 0.009 |
| | | 14mer | 336 | 0 | 6 | 0.018 |
| | CTP-γ2 | 13mer | 344 | 0 | 3 | 0.009 |
| | | 14mer | 336 | 0 | 5 | 0.015 |
| | CTP-γ3 | 13mer | 344 | 0 | 2 | 0.006 |
| | | 14mer | 336 | 0 | 4 | 0.012 |

**TABLE 20**

| Rhesus macaque | | | | | | |
|---|---|---|---|---|---|---|
| Group | CTP | scan length | Total | SB | WB | (SB+WB)/ Total |
| CTP γ | CTP-γ1 | 13mer | 774 | 4 | 17 | 0.027 |
| | | 14mer | 756 | 6 | 25 | 0.041 |
| | CTP-γ2 | 13mer | 774 | 4 | 17 | 0.027 |
| | | 14mer | 756 | 6 | 26 | 0.042 |
| | CTP-γ3 | 13mer | 774 | 4 | 19 | 0.030 |
| | | 14mer | 756 | 6 | 26 | 0.042 |

**TABLE 21**

| Mouse | | | | | | |
|---|---|---|---|---|---|---|
| Group | CTP | scan length | Total | SB | WB | (SB+WB)/ Total |
| CTP γ | CTP-γ1 | 13mer | 258 | 1 | 11 | 0.047 |
| | | 14mer | 252 | 1 | 14 | 0.060 |
| | CTP-γ2 | 13mer | 258 | 1 | 11 | 0.047 |
| | | 14mer | 252 | 1 | 14 | 0.060 |
| | CTP-γ3 | 13mer | 258 | 1 | 12 | 0.050 |
| | | 14mer | 252 | 1 | 14 | 0.060 |

**TABLE 22**

| BoLA | | | | | | |
|---|---|---|---|---|---|---|
| Group | CTP | scan length | Total | SB | WB | (SB+WB)/ Total |
| CTP γ | CTP-γ1 | 13mer | 258 | 0 | 0 | 0.000 |
| | | 14mer | 252 | 0 | 1 | 0.004 |
| | CTP-γ2 | 13mer | 258 | 0 | 0 | 0.000 |
| | | 14mer | 252 | 0 | 1 | 0.004 |
| | CTP-γ3 | 13mer | 258 | 0 | 0 | 0.000 |
| | | 14mer | 252 | 0 | 0 | 0.000 |

**TABLE 23**

| Pig | | | | | | |
|---|---|---|---|---|---|---|
| Group | CTP | scan length | Total | SB | WB | (SB+WB)/ Total |
| CTP γ | CTP-γ1 | 13mer | 129 | 0 | 0 | 0.000 |
| | | 14mer | 126 | 0 | 1 | 0.008 |
| | CTP-γ2 | 13mer | 129 | 0 | 0 | 0.000 |
| | | 14mer | 126 | 0 | 1 | 0.008 |
| | CTP-γ3 | 13mer | 129 | 0 | 0 | 0.000 |
| | | 14mer | 126 | 0 | 1 | 0.008 |

### <Example 14> Cloning of coronavirus infectious disease COVID-19 therapeutic protein CTP gamma

### 1. Strain and medium

All chemicals used for gene cloning were of analytical grade. Completed clones were transformed using a DH5α E. coli strain for proliferation and screened with LB medium in which ampicillin (LPS, 100 µg/ml) was added. Cell culture was performed at 37°C with stirring involved.

### 2. Construction of plasmids

After converting the amino acid sequence of the protein designed in silico into nucleotides using the Sequence Manipulation Suite (https://www.bioinformatics.org/sms2/rev_trans.html) tool, DNA sequences were determined by comparing with the sequence present in hACE2. The synthesized DNA fragment (Bioneer, gene synthesis) was amplified by PCR using a forward primer 5'-GGAGATATACATATGAAAGCTAGTTCACTTGCTGATAA(CTP-γ1), 5'-GGAGATATACATATGAAAGAAAGTTCACTTGCTGATAATG(CTP-γ2), and 5'-GGAGATATACATATGAAAGCTAGTGAACTTGCTGATAA(CTP-γ3) as well as a common reverse primer 5'-GTGGTGCTCGAGCCTGAAGTCGCCCTTCC, and then inserted into a pET-21a vector treated with restriction enzymes Nde I and Xho I by ligation independent cloning using EZ-Fusion^{™} HT Cloning Kit (Engnomics).

### 3. Experiment result

The therapeutic protein CTP gamma of the present disclosure is a therapeutic protein of a new amino acid sequence that neutralizes or inhibits the S1-RBD protein from binding to the hACE2 protein, wherein the amino acid sequence of the therapeutic protein CTP gamma designed in silico is shown in Table 24.

**TABLE 24**

| Group γ | |
|---|---|
| CTP name | Sequence |
| CTP-γ1 | MKASSLADNVYNTNITKENVQNMNEEQAKTFLDKFNHEAEDLF YQSSGLGKGDFR (SEQ ID NO: 7) |
| CTP-γ2 | MKESSLADNVYNTNITKENVQNMNEEQAKTFLDKFNHEAEDLF YQSSGLGKGDFR (SEQ ID NO: 8) |
| CTP-γ3 | MKASELADNVYNTNITKENVQNMNEEQAKTFLDKFNHEAEDLF YQSSGLGKGDFR (SEQ ID NO: 9) |

### <Example 15> Expression and purification of coronavirus infectious disease COVID-19 therapeutic protein CTP gamma

### 1. Protein expression and purification materials

All chemicals used for protein expression and purification were of analytical grade. Ampicillin, 1-thio-γ-d-galactopyranoside (IPTG), sodium phosphate-monobasic, sodium phosphate-dibasic, and TRIS used for protein expression and purification were purchased from LPS (Daejeon, Korea), Coomassie brilliant blue R-250, 2-mercaptoethanol, chloramphenicol, and imidazole were purchased from Biosesang (Seongnam, Korea), sodium chloride was purchased from Duksan (Daejeon, Korea), and all columns were purchased from GE Healthcare (Piscataway, NJ). Phenylmethanesulfonyl fluoride (PMSF), trizma hydrochloride, and Amicon Ultra-15 Centrifugal Filter Units used herein were purchased from Millipore (Billerica, MA).

### 2. Protein expression and purification instruments

Shake incubator (NBS/Innova 42R) and high-performance high-capacity centrifuge (Beckman Coulter. Inc./AVANTI JXN-26) were used for protein expression, and protein high-speed separation system (GE Healthcare/AKTA Pure M and Start System), protein high-speed separation system (GE HealthcareIFPLC Accessory System), ultrasonicator (Q700-Sonicator), and refrigerated centrifuge (Epoendorf-5810R) were used to isolate and purify proteins.

### 3. Expression and culture of therapeutic protein CTP gamma

The gene cloned in the pET-21a vector was used for transformation for expression using a BL21 (DE3) RIL E. coli strain, and cell culture was performed at 37°C using LB medium in which ampicillin (100 mg/ml) and chloramphenicol (50 mg/ml) were added. After culture to meet OD₆₀₀ value of 0.6-0.8 (OD₆₀₀=0.6~0.8), 1M IPTG was added to a final concentration of 0.5 mM, followed by expression at 37°C for 4 hours. A high-performance high-capacity centrifuge (Beckman Coulter. Inc./AVANTI JXN-26) set at 7000 RPM, 4°C was used to harvest the cells for 20 minutes to be used immediately or stored at -80°C.

### 4. Isolation and purification of therapeutic protein CTP gamma

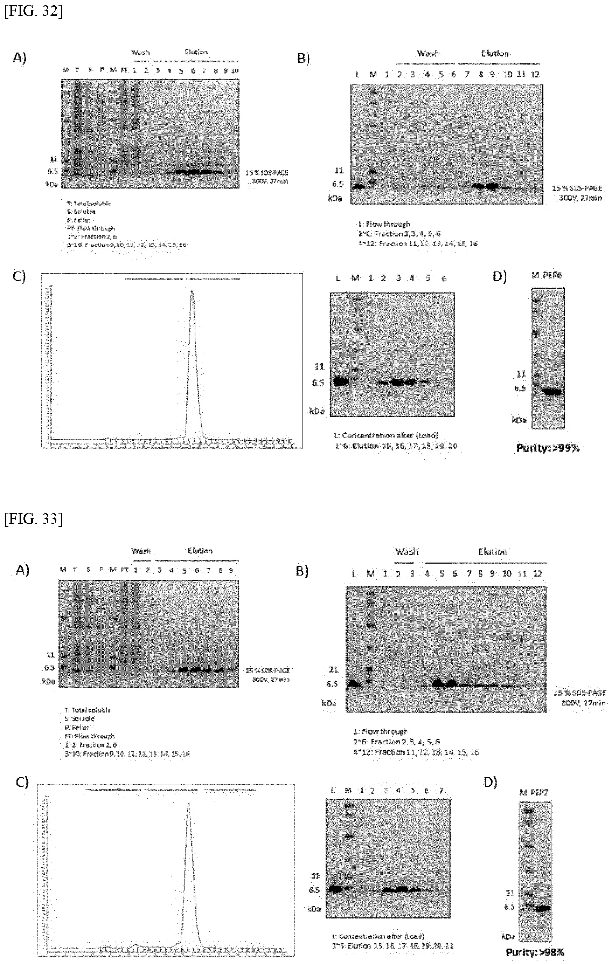

After dissolving in 10 ml of lysis buffer (pH 8.0, 50 mM Tris-HCl, 500 mM NaCl, 1 mM PMSF, 0.25% Tween-20 (v/v)) based on 1 g of cells, the cells were disrupted using a sonicator (Q700-Sonicator). In order to isolate the solution and the pellet, a high-speed centrifuge set at 18000 RPM and 4°C was used for 30 minutes. A 0.45 µm syringe filter was used for removal of impurities in the aqueous solution. The solution from which impurities were removed was poured through a HisTrap HP 5ml (GE Healthcare) column stabilized with wash buffer (pH 8.0, 50 mM Tris-HCl, 500 mM NaCl), and then elution buffer (pH 8.0, 50 mM Tris-HCl, 500 mM NaCl, 500 mM imidazole) was set to 0 to 100% gradient elution. As a result, it was found that the therapeutic protein CTP gamma was isolated with imidazole at a concentration of 100 to 300 mM. The isolated therapeutic protein CTP gamma was subjected to buffer exchange (pH 8.0, 20 mM Tris-HCl) in a cold room for 15 hours. After pouring the NaCl-removed CTP gamma solution through the HiTrap Q HP 5ml (GE Healthcare) column stabilized with binding buffer (pH 8.0, 20 mM Tris-HCl), elution buffer (pH 8.0, 20 mM Tris-HCl, 1.0 M NaCl) was set from 0 to 100% gradient elution. As a result, it was found that the therapeutic protein CTP gamma was isolated with NaCl at a concentration of 120 to 250 mM. Finally, therapeutic protein CTP gamma having a dimer structure was purely isolated and purified using a HiLoad Superdex 75 16/600 column stabilized with pH 7.4, 10 mM phosphate buffer, and 150 mM NaCl. Quantification was performed at A₂₈₀ₙₘ using NanoDrop, and, as a result of calculating the purity using the ImageJ program, high purity (>96%) therapeutic protein CTP gamma protein was obtained (FIGS. 32 to 34).

### <Example 16> Mass spectrometry of coronavirus infectious disease COVID-19 therapeutic protein CTP gamma

### 1. Mass spectrometry analysis

Mass spectrometry was used to identify the total mass and information on the amino acid sequence of the protein. Mass spectrometry of total proteins was performed using a high-performance matrix laser desorption ionization mass spectrometer (Bruker, UltrafleXtreme MALDI TOF/TOF). Hybrid triple quadrupole linear ion trap mass spectrometer (Sciex, QTRAP) and quadrupole orbitrap mass spectrometer (Thermo scientific, Q Exactive PLUS) were used for amino acid sequence analysis for the protein and micro liquid chromatography (Sciex, M5 MicroLC) was used for QTRAP, and nano-micro liquid chromatography (Waters, ACQUITY UPLC M-Class system) was used for Q Exactive PLUS.

### 2. Sample preparation for MALDI TOF and analysis of intact proteins using MALDI TOF

For total mass spectrometry of ACE2, RBD, and CTP gamma proteins, 10 µg of protein obtained by removing low-molecular compounds using Microcon centrifugal filter (Merck, MRCPRT010) and sinapinic acid (Bruker, 8201345) matrix were mixed in a 1:1 ratio, and the mixture was then placed on a target plate to dry and subjected to the flexcontrol program of the high-performance matrix laser desorption ionization mass spectrometer. The mass value was normalized using the Starter kit for MALDI-TOF MS (Bruker, 8208241), and the spectrum was obtained with an average of 1000 laser shots based on the method provided by flexcontrol. The result values were derived from each spectral data using the flexanalysis program.

### 3. Sample preparation for LC-MS/MS analysis

For amino acid sequence analysis of a single protein, 20 µg of RBD, hACE2, and CTP gamma proteins were mixed with 100 mM triethylammonium bicarbonate (Sigma, T7408-100ML) and 2% sodium dodecyl sulfate (Duksan, 6645) to a specific concentration. After adding 10 mM dithiothreitol (BIO-RAD, 161-0611) and treating at 56°C for 30 minutes, 20 mM iodoaetamide (Sigma, T1503) was added and shielded from light, followed by treatment at room temperature for 30 minutes. Then, using S-trap mini columns (PROTIFI, C02-mini-80), low molecular weight compounds were removed, and 2 µg of proteases including trypsin (Promega, V5111) and Glu-C (Promega, V1651) were added, followed by enzymatic degradation at 37°C for 16 hours. After termination of the reaction, isolation was performed with a centrifuge, drying was followed under vacuum, and 0.1% trifluoroacetic acid was added to prepare a sample. In addition, deglycosylation experiments were added for RBD and ACE2 proteins. For deglycosylation experiment, 100 mM triethylammonium bicarbonate and 2 µg of PNGase F (Promega, V4831) were added to the protease-treated sample, followed by enzymatic degradation at 37°C for 4 hours. After termination of the reaction, the proteins were isolated by centrifuge using S-trap mini columns. Vacuum drying was followed, and then 0.1% trifluoroacetic acid was added to prepare a sample. For the RBD and hACE2 proteins, a quadrupole orbitrap mass spectrometer was used, and experiments proceeded via nano-micro liquid chromatography. For the CTP gamma protein, a hybrid triple quadrupole linear ion trap mass spectrometer was used, and experiments proceeded using micro-liquid chromatography.

### 4. LC-MS/MS Analysis: ACQUITY UPLC M-Class system - Q Exactive PLUS

All solvents used for liquid chromatography were of HPLC grade. Water (Duksan, 7732-18-5) and acetonitrile (Honeywell, AH015-4) were used as solvents, and dimethyl sulfoxide (Sigma, 472301-1L), trifluoroacetic acid (Thermo scientific, 28904), and formic acid (Thermo scientific, 28905) were used as a medium in the experimental solution. Nano-micro liquid chromatography was composed of solvent A (0.1% formic acid, 5% dimethyl sulfoxide in water) and solvent B (0.1% formic acid, 5% dimethyl sulfoxide in 80% acetonitrile). Acclaim^{™} PepMap^{™} 100 C18 LC Column (Thermo scientific, 164197) was used as a trapping column, and EASY-Spray^{™} HPLC Columns (Thermo scientific, ES803A) as an analytical column. Liquid chromatography was performed for 5 minutes in the presence of 95% solvent A and 5% solvent B at a flow rate of 8 µl/min in the trapping step after injecting 0.5 µg of the sample. In the analytical step, the flow rate was 250 nl/min. After 5 minutes under the conditions of 95% solvent A and 5% solvent B, the samples were analyzed by gradually increasing the rate of solvent B from 5% to 40% for 140 minutes, the column was activated for 15 minutes after increasing to 95% for 5 minutes again, and re-equilibration was followed by decreasing the rate of solvent B to 5% for 5 minutes and maintaining the state for 40 minutes. The mass values of quadrupole orbitrap mass spectrometer were calibrated by Pierce^{™} LTQ Velos ESI Positive Ion Calibration Solution (Thermo scientific, 88323). The analysis was conducted via full-MS and data dependent MS/MS methods. The calculated data was obtained using a proteome discoverer (Thermo scientific).

### 5. LC-MS/MS Analysis: M5 MicroLC - QTRAP

The column used for the analysis was a Kinetex 2.6u XB-C18 100A (Phenomenex, 00B-4496-AC). Micro liquid chromatography was composed of solvent A (0.1% formic acid in water) and solvent B (0.1% formic acid in acetonitrile). The liquid chromatography was performed at a flow rate of 20 µl/min after injecting 1.5 µg of the sample. After 5 minutes under the conditions of 95% solvent A and 5% solvent B, the samples were analyzed by gradually increasing the rate of solvent B from 5% to 40% for 45 minutes, the column was activated for 4 minutes after increasing to 95% for 5 minutes again, and re-equilibration was followed by decreasing the rate of solvent B to 5% for 3 minutes and maintaining the state for 3 minutes. The mass values of hybrid triple quadrupole linear ion trap mass spectrometer were calibrated by standard chemical kit (Sciex, 4406127). The analysis was conducted using Q3 MS and enhanced product ion method using information dependent acquisition. For the calculated data, the wiff file was converted to the mgf file using convert MS (proteowizard.sourceforge.net), and the results were obtained using searchGUI and peptide-shaker (compomics.github.io).

### 6. Results of intact protein mass spectrometry

The mass of the intact protein was measured using MALDI-TOF. The mass of RBD was 30 kDa. In addition to 30kDa, the peak was a dimer, appeared along with a 60kDa peak, a monomer with a charge (z) of 2 (m/z=15kDa), and a trimer with a charge (z) of 2 (m/z=45kDa). Considering that the mass of RBD calculated from the protein sequence is 25.9 kDa, it may be suggested that glycosylation caused the change in mass of 4.1 kDa. Similarly, the mass of hACE2 was measured to be about 102 kDa, and a dimer and a peak with a charge of 2 also appeared together. Considering that the mass of hACE2 calculated from the protein sequence is 84.4 kDa, it is suggested that the mass change of about 17.5 kDa was caused by glycosylation.

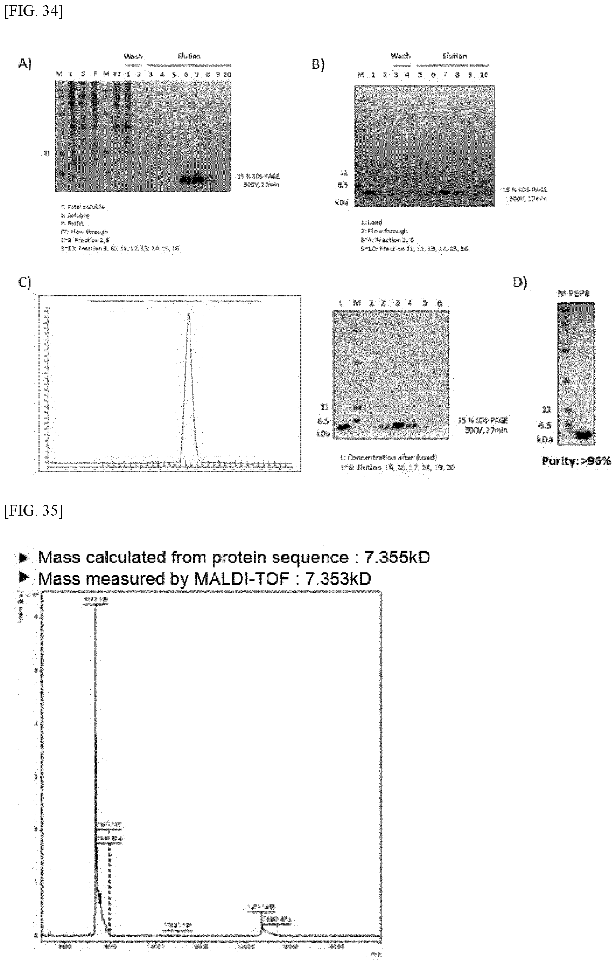

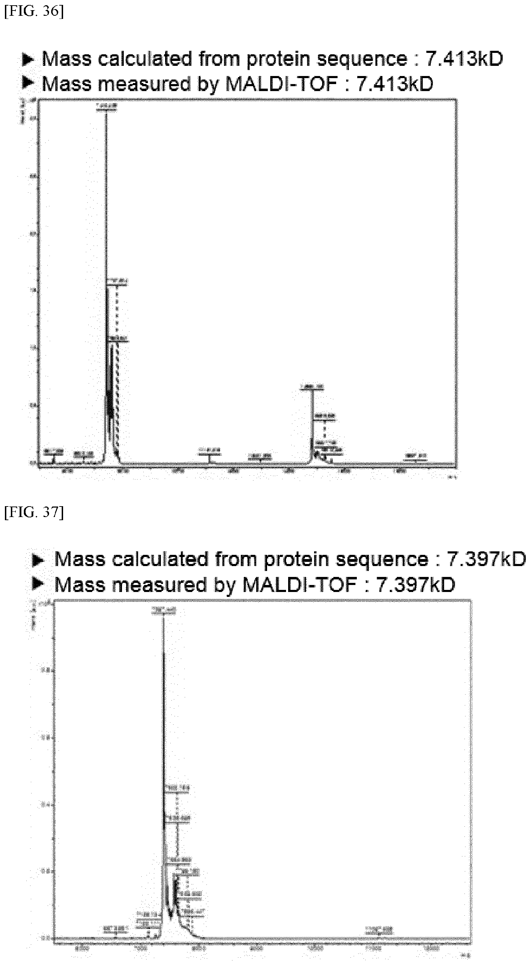

CTP-γ1 was measured to be 7.353kD, almost equal to the mass calculated from the sequence (FIG. 35), CTP-γ2 was measured to be 7.413kD, almost equal to the mass calculated from the sequence (FIG. 36), and CTP-γ3 was measured to be 7.397kD, almost equal to the mass calculated from the sequence (Fig. 37).

### 7. Identification of protein sequences

LC-MS/MS analysis was performed to identify the sequences of RBD, hACE2, and CTP gamma. Each protein was subjected to protein analysis using proteases such as trypsin and Glu-C, wherein, for RBD and hACE2, N-glycosylated Asn was found by searching for amino acid residues in which Asn was changed to Asp by enzymatic activity of PNGase F.

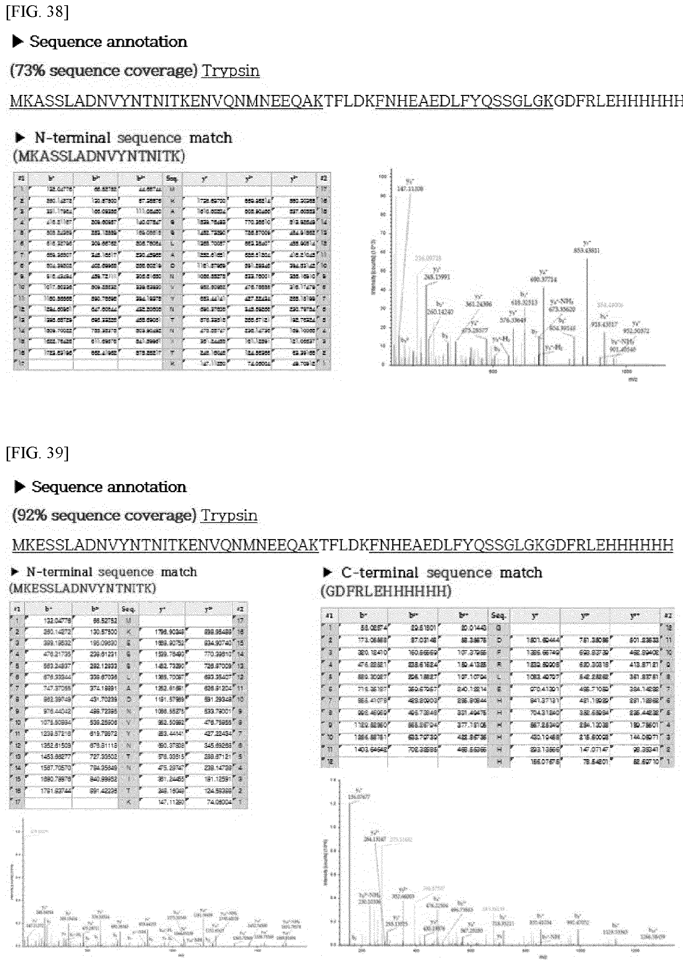

The results of MS/MS analysis of CTP gamma having the N, C-terminus identified are shown in FIGS. 38 to 40. Sequence analysis of the N, C-terminus is one of the means for identifying the state of the protein, and if the terminus of the protein is not identified, it may be considered as being degraded due to a problem in the preservation state of the protein. In the case of RBD and hACE2, the terminus of the proteins tends not to be identified (ID), so unless inaccuracy in the sequence or the effect of PTM matters, they are easily degraded, so care must be taken for preservation.

### <Example 17> Secondary structural stability and function/efficacy verification of coronavirus infectious disease COVID-19 therapeutic protein CTP gamma

### 1. Circular dichroism (CD) experiment

Far UV circular dichroism was measured for RBD (PBS buffer), hACE2 (PBS buffer), and CTP gamma. A JASCO-1500 machine was used with the protein concentration of 0.2 mg/ml and a quartz cell of 0.1 cm at a temperature of 20°C. Measurements were performed at a measurement speed of 20 nm/min, a bandwidth of 5 nm, and a digital integration time (D.I.T) of 4 seconds at a far UV wavelength of 190-250 nm. A graph was drawn by subtracting the data obtained by 5 times measurement for the protein by the data obtained by 5 times measurement for the buffer.

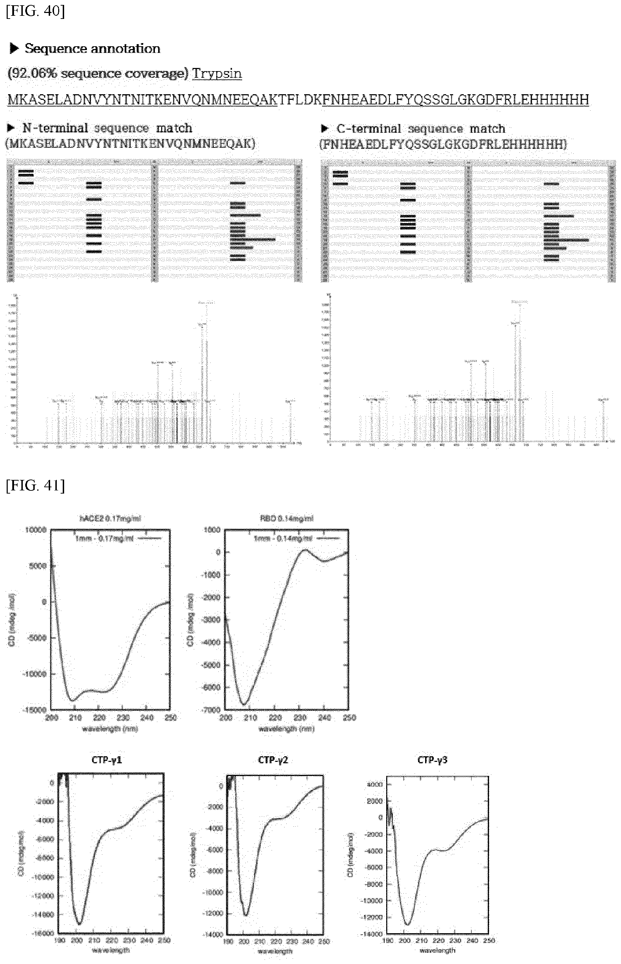

As a result, it was found that hACE2 is a protein having α-helix as a secondary structure, and RBD is a protein having a beta sheet as a secondary structure. It was found that CTP gamma has both a helical structure and a coil structure (FIG. 41).

### 2. Microscale thermophoresis (MST) experiment

Fluorescence was attached to 100 µL of 8 µM RBD (PBS buffer with 0.05% Tween-20 added) using Monolith Protein Labeling Kit RED-NHS 2nd Generation. Ligand (hACE2 (PBS buffer) and CTP gamma were prepared by 10 µL each with 16 concentrations from 16 µM to 0.488 nM using a 1:1 serial dilution. A mixed solution of 15 nM RBD and 8 µM-0.244 nM ligand was prepared by diluting the fluorescence-attached RBD to 30 nM and adding 10 µL of the RBD to each ligand of 16 concentrations. MST was measured 5 times for each ligand using a Monolith NT.115 machine with 60% excitation power and medium MST power. Kd (dissociation constant) value was calculated using Kdmodel of MO affinity analysis program provided by Monolith company. Experimental results having the median Kd value derived from 5 experiments are shown in a graph.

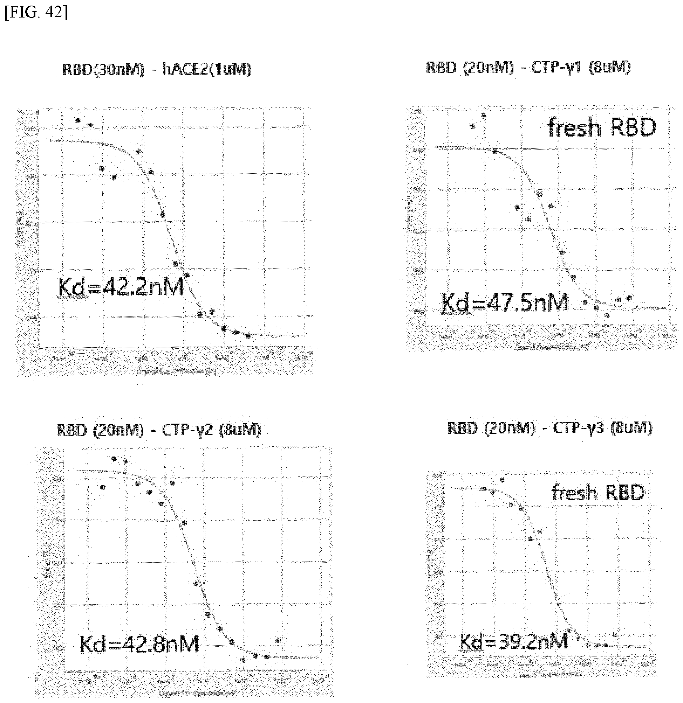

As a result, a change in concentration-dependent fluorescence intensity was observed, indicating that both hACE2 and CTP gamma bind to RBD. The Kd values representing the binding strength were 47.5 nM for CTP-γ1, 42.8 nM for CTP-γ2, 39.2 nM for CTP-γ3, and 42.2 nM for hACE2 (FIG. 42). The Kd values of RBD and hACE2 are similar to that of 34.6 nM measured by using BLI (biolayer interferometry) and SPR (Surface Plasmon Resonance) in the Science paper (Wrapp et al, Science 367, 1260-1263 (2020)).

### <Example 18> Toxicity test for coronavirus infectious disease COVID-19 therapeutic protein CTP gamma

### 1. Cell culture for toxicity analysis of therapeutic protein CTP gamma

The cell line used in the present disclosure was obtained from the Korea Cell Line Bank (KCLB, Seoul, Korea). Used as a culture medium for the human embryonic kidney cell line HEK293T, the human liver cancer cell line HepG2, and the human lung cancer cell line A549 was Dulbecco's modified eagle medium (DMEM, Hyclone, USA) supplemented with 10% fetal bovine serum (FBS, Hyclone, USA) and 1% antibiotic (penicillin-streptomysin; P/S, Gibco, USA), and human microglia HMC-3 was cultured in Minimum Essential Media (MEM, Gibco, USA) supplemented with 10% fetal bovine serum and 1% antibiotic (penicillin-streptomycin). Used as a culture medium for human normal lung cell line MRC-5 was Minimum Essential Media (MEM) supplemented with 10% fetal bovine serum, 1% antibiotic (penicillin-streptomycin) and 25 µM hydroxyethyl piperazine ethane sulfonic acid (HEPES, Hyclone, USA). For the human kidney cancer cell line Caki-1, McCoy's 5A medium (Gibco, USA) supplemented with 10% fetal bovine serum and 1% antibiotics (penicillin-streptomycin) was used as a culture medium. All cell lines used were cultured in an incubator controlled to a temperature of 37°C in the presence of 5% CO₂.

### 2. Cytotoxicity analysis for therapeutic protein CTP gamma

The HEK293T, HepG2, HMC-3, MRC-5, A549, and Caki-1 cell lines were dispensed into 96-well cell culture plates in the count of 3×10³, 6×10³, 5×10³, 6×10³, 5×10³, and 5×10³ cells/well, respectively, and then stabilized in an incubator controlled at a temperature of 37°C in the presence of 5% CO₂ for 24 hours. Then, a starvation culture medium in which the FBS content was reduced from 10% to 1% in the culture medium composition for each cell line was prepared and replaced with a starvation culture medium, followed by starvation for 16 hours. Thereafter, 1 fg, 10 fg, 100 fg, 1 pg, 10 pg, 100 pg, 1 ng, 10 ng, 100 ng, 1 µg, and 10 µg of CTP gamma were added to 100 µl of the culture medium, respectively, and the mixture was treated to the cell line, followed by culture for 24 hours and 48 hours. Viability of the cell lines was measured using Cell Counting Kit-8 (CCK-8, Dojindo, Kumamoto, Japan). After adding 10 µl of CCK-8 reagent per 100 µl of culture medium and culturing for 1 hour in the incubator controlled at a temperature of 37°C in the presence of 5% CO₂, the optical density (O.D.) value was measured by absorbance at 450 nm using a SpectraMax iD3 microplate reader (Molecular Devices, San Jose, CA, USA), and the derived value was converted into a percentage.

### 3. Effect of therapeutic protein CTP gamma on cell viability of each cell line

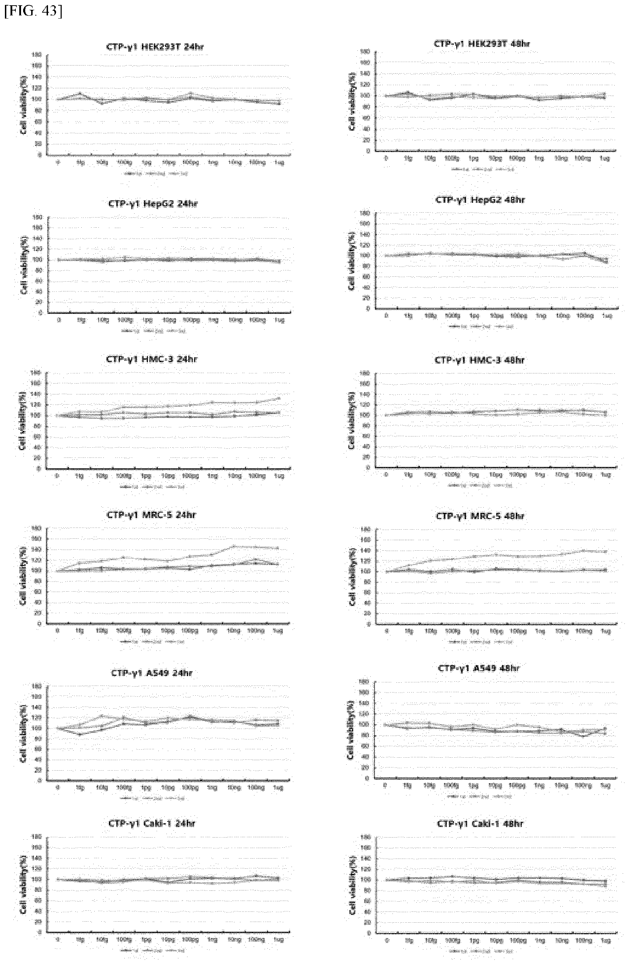

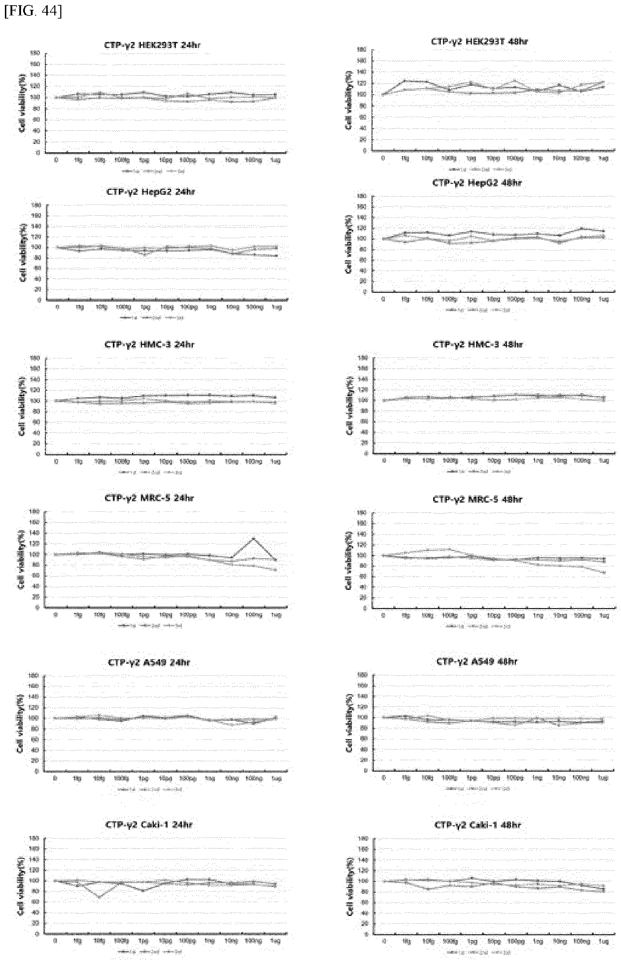

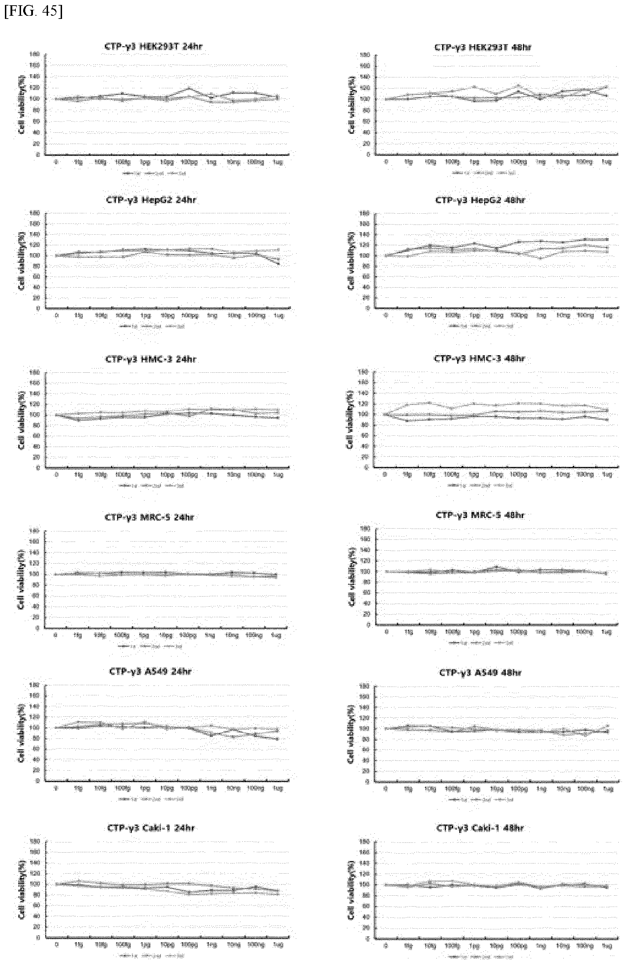

In the present disclosure, a CCK-8 assay was performed to check the toxicity of CTP gamma in 6 cell lines including HEK293T, HepG2, HMC-3, MRC-5, A549, and Caki-1 on the cell survival of each cell line. As a result, after treatment with CTP gamma for 24 hours, when the cell viability of each cell line was checked, no significant change was observed in all 6 cell lines compared to the control group not treated with CTP gamma. In addition, after treatment with CTP gamma for 48 hours, when the cell viability of each cell line was checked, no significant change was observed in all 6 cell lines (FIGS. 43 to 45). Overall, the cytotoxicity analysis results proposed applicability of CTP gamma for preventing and treating coronavirus by finding the nontoxicity and safety of the CTP gamma.

### <Example 19> 3D structural analysis of a protein of coronavirus infectious disease COVID-19 therapeutic protein CTP delta

### 1. Preparation of a 3D structure of a protein

A binding structure of hACE2-RBD registered in Protein Data Bank (PDB, https://www.rcsb.org/) (PDB ID: 6M0J) was used. The structure of a P6 moiety was modeled using MODELLER based on the binding structure of hACE2-RBD using the sequence of 22-44-G-351-357 of hACE2.

### 2. Molecular dynamic simulation

For the modeled hACE2-RBD complex, P6-RBD complex, CTP delta monomer-RBD complex, P6, and CTP delta monomer, molecular dynamics simulations to which the ff14SB force field was applied were performed using the AMBER18 package. The simulation was performed with the pmemd.cuda program included in AMBER18, and an octahedron TIP3P water box with a length of 15 Å added to the outer portion of the prepared protein was applied for the initial structure for the simulation. A periodic boundary condition was applied based on the water box, and Na+ and Cl- ions were added to neutralize the net charge. The particle-mesh Ewald (PME) method was applied for electromagnetic force of long distance based on a distance of 9 Å. The SHAKE algorithm to fix the distance of a covalent bond of hydrogen molecules was used, and the simulation was performed with a time step of 2 fs/step. First, a 5000-step minimization simulation was performed by applying 0.5 kcal/mol of a position restraint to the backbone structure of the protein, and a heating simulation was performed for 25 ps under NVT ensemble conditions from 10K to 300K by applying 0.1 kcal/mol of a position restraint. Equilibrium simulations were performed under NPT ensemble conditions at a temperature of 300K for 1 ns. The production run was performed under NPT ensemble conditions at a temperature of 300K and a pressure of 1 bar, and the simulation was performed for 500 ns for a complex with RBD and 1 µs for CTP delta alone. Simulations were performed with 5 independent trajectories for each case.

The binding energy of RBD and CTP delta were calculated by the MMGBSA algorithm using simulation snapshots within 300 to 500 ns, and entropy was calculated via normal mode analysis. The MMPBSA.py program in the AMBER18 package was used to calculate the binding free energy.

### 3. Structure of CTP delta

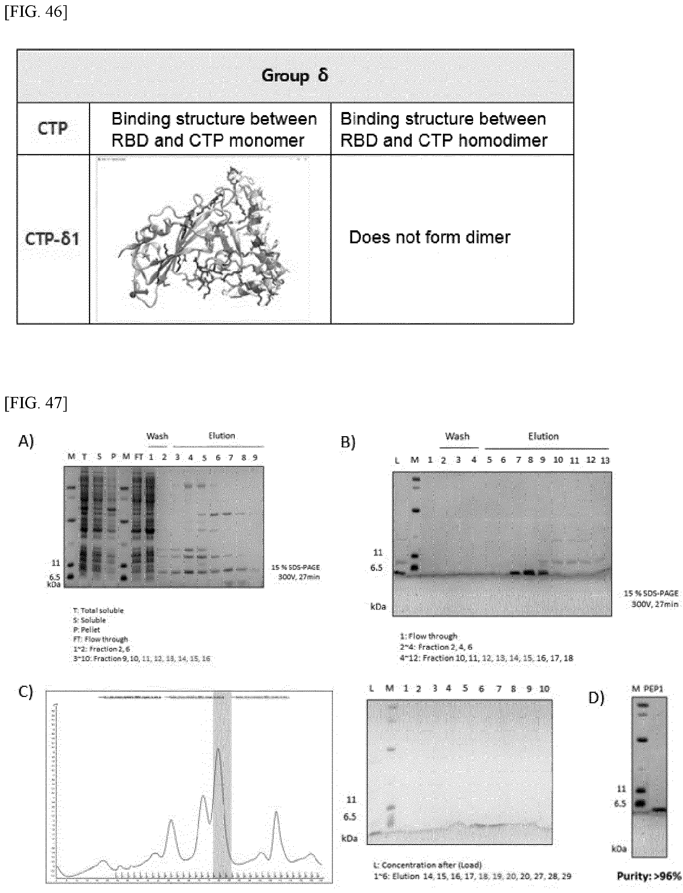

Modeled CTP-61 has a structure of an HTH 46AA-type. When the structural ensemble of the modeled structure of the monomer form in a solution was analyzed through molecular dynamics simulation, the structural patterns shown in FIG. 46 were observed.

CTP-61 is a therapeutic protein with a flexible turn structure in which E3, D6, K7, K9, and E11 to interact with R454, K458, D467, and E471 of RBD are positioned by adding a Helix-turn-Helix structure to the N-terminal portion. Strong binding with RBD is possible, but structural stability is lowered.

### 4. Prediction of binding free energy of CTP delta-RBD

Binding energy and binding free energy were calculated to determine how well CTP delta binds to RBD (Table 25). Using molecular dynamics simulation, structural ensembles in which hACE2, P6, and CTP deltas bind to RBD, respectively, were collected, and the binding energy (ΔE) and binding free energy (ΔG) from the structural ensemble were analyzed using the MMGBSA algorithm.

It was found that CTPs have negative binding free energy and are stable when bound to RBD, bind with stronger energy (ΔE) than hACE2 and P6 in simulations due to interaction with a new epitope, and also bind with similar or stronger free energy (ΔG).

**TABLE 25**

| Group | CTP | RBD + CTP monomer | | RBD + CTP dimer | |
|---|---|---|---|---|---|
| | | ΔE | ΔG | ΔE | ΔG |
| - | hACE2 | -74.0327 | - | - | - |
| - | P6 | -63.9558 | -11.0060 | - | - |
| CTP δ | CTP-δ 1 | -60.0639 | -5.3909 | - | - |

### 5. In-silico immunogenicity analysis of CTP delta

In-silico immunogenicity was analyzed using the globally well-established NetMHC-4.0 server (http://www.cbs.dtu.dk/services/NetMHC/). For the amino acid sequence of CTP delta, prediction was made on the binding of peptide-MHC class I for 81 MHC alleles of each different individual (human) and 41 alleles of animals (monkey, cattle, pig, and mouse) in the NetMHC4.0 server. By searching in peptide units of 13-14 amino acids in length, the total number of searched peptides and the number of peptides expected to have strong binding (SB) and weak binding (WB) thereamong were counted, and the ratio was calculated by (SB+WB)/Total to verify immunogenicity.

It indicates that the lower the ratio to the total (SB+WB)/Total is, the less the antigen-antibody reaction occurs with other proteins when the neutralizing therapeutic protein of the present disclosure is injected into the body (Tables 26 to 31).

**TABLE 26**

| Human | | | | | | |
|---|---|---|---|---|---|---|
| Group | CTP | scan length | Total | SB | WB | (SB+WB)/ Total |
| CTP δ | CTP-δ1 | 13mer | 2754 | 13 | 23 | 0.013 |
| | | 14mer | 2673 | 17 | 28 | 0.017 |

**TABLE 27**

| Chimpanzee | | | | | | |
|---|---|---|---|---|---|---|
| Group | CTP | scan length | Total | SB | WB | (SB+WB)/ Total |
| CTP δ | CTP-δ1 | 13mer | 272 | 0 | 1 | 0.004 |
| | | 14mer | 264 | 0 | 2 | 0.008 |

**TABLE 28**

| Rhesus macaque | | | | | | |
|---|---|---|---|---|---|---|
| Group | CTP | scan length | Total | SB | WB | (SB+WB)/ Total |
| CTP δ | CTP-δ1 | 13mer | 612 | 4 | 17 | 0.034 |
| | | 14mer | 594 | 6 | 21 | 0.045 |

**TABLE 29**

| Mouse | | | | | | |
|---|---|---|---|---|---|---|
| Group | CTP | scan length | Total | SB | WB | (SB+WB)/ Total |
| CTP δ | CTP-δ1 | 13mer | 204 | 1 | 7 | 0.039 |
| | | 14mer | 198 | 2 | 7 | 0.045 |

**TABLE 30**

| BoLA | | | | | | |
|---|---|---|---|---|---|---|
| Group | CTP | scan length | Total | SB | WB | (SB+WB)/ Total |
| CTP δ | CTP-δ1 | 13mer | 204 | 0 | 0 | 0.000 |
| | | 14mer | 198 | 0 | 0 | 0.000 |

**TABLE 31**

| Pig | | | | | | |
|---|---|---|---|---|---|---|
| Group | CTP | scan length | Total | SB | WB | (SB+WB)/ Total |
| CTP δ | CTP-δ1 | 13mer | 102 | 0 | 0 | 0.000 |
| | | 14mer | 99 | 0 | 1 | 0.010 |

### <Example 20> Cloning of coronavirus infectious disease COVID-19 therapeutic protein CTP delta

### 1. Strain and medium

All chemicals used for gene cloning were of analytical grade. Completed clones were transformed using a DH5α E. coli strain for proliferation and screened with LB medium in which ampicillin (LPS, 100 µg/ml) was added. Cell culture was performed at 37°C with stirring involved.

### 2. Construction of plasmids

After converting the amino acid sequence of the protein designed in silico into nucleotides using the Sequence Manipulation Suite (https://www.bioinformatics.org/sms2/rev_trans.html) tool, DNA sequences were determined by comparing with the sequence present in hACE2. The synthesized DNA fragment (Bioneer, gene synthesis) was amplified by PCR using a forward primer 5'-GGAGATATACATATGGGCGAATTTGCGGATAAGTTG and a reverse primer 5'-GTGGTGCTCGAGCCTGAAGTCGCCCTTCC and then inserted into a pET-21a vector treated with restriction enzymes such as Nde I and Xho I by ligation independent cloning using EZ-Fusion^{™} HT Cloning Kit (Engnomics).

### 3. Experiment result

The therapeutic protein CTP delta of the present disclosure is a therapeutic protein of a new amino acid sequence that neutralizes or inhibits the S1-RBD protein from binding to the hACE2 protein, wherein the amino acid sequence of the therapeutic protein CTP delta designed in silico is shown in Table 32.

**TABLE 32**

| Group δ | |
|---|---|
| CTP name | Sequence |
| CTP-δ1 | MGEFADKLKNELAKTEEQAKTFLDKFNHEAEDLFYQSSGLGKG DFR (SEQ ID NO: 10) |

### <Example 21> Expression and purification of coronavirus infectious disease COVID-19 therapeutic protein CTP delta

### 1. Protein expression and purification materials

All chemicals used for protein expression and purification were of analytical grade. Ampicillin, 1-thio-δ-d-galactopyranoside (IPTG), sodium phosphate-monobasic, sodium phosphate-dibasic, and TRIS used for protein expression and purification were purchased from LPS (Daejeon, Korea), Coomassie brilliant blue R-250, 2-mercaptoethanol, chloramphenicol, and imidazole were purchased from Biosesang (Seongnam, Korea), sodium chloride was purchased from Duksan (Daejeon, Korea), and all columns were purchased from GE Healthcare (Piscataway, NJ). Phenylmethanesulfonyl fluoride (PMSF), trizma hydrochloride, and Amicon Ultra-15 Centrifugal Filter Units used herein were purchased from Millipore (Billerica, MA).

### 2. Protein expression and purification instruments

Shake incubator (NBS/Innova 42R) and high-performance high-capacity centrifuge (Beckman Coulter. Inc./AVANTI JXN-26) were used for protein expression, and protein high-speed separation system (GE Healthcare/AKTA Pure M and Start System), protein high-speed separation system (GE Healthcare/FPLC Accessory System), ultrasonicator (Q700-Sonicator), and refrigerated centrifuge (Epoendorf-5810R) were used to isolate and purify proteins.

### 3. Expression and culture of therapeutic protein CTP delta

The gene cloned in the pET-21a vector was used for transformation for expression using a BL21 (DE3) RIL E. coli strain, and cell culture was performed at 37°C using LB medium in which ampicillin (100 mg/ml) and chloramphenicol (50 mg/ml) were added. After culture to meet OD₆₀₀ value of 0.6-0.8 (OD₆₀₀=0.6~0.8), 1M IPTG was added to a final concentration of 0.5 mM, followed by expression at 37°C for 4 hours. A high-performance high-capacity centrifuge (Beckman Coulter. Inc./AVANTI JXN-26) set at 7000 RPM, 4°C was used to harvest the cells for 20 minutes to be used immediately or stored at -80°C.

### 4. Isolation and purification of therapeutic protein CTP delta

After dissolving in 10 ml of lysis buffer (pH 8.0, 50 mM Tris-HCl, 500 mM NaCl, 1 mM PMSF, 0.25% Tween-20 (v/v)) based on 1 g of cells, the cells were disrupted using a sonicator (Q700-Sonicator). In order to isolate the solution and the pellet, a high-speed centrifuge set at 18000 RPM and 4°C was used for 30 minutes. A 0.45 µm syringe filter was used for removal of impurities in the aqueous solution. The solution from which impurities were removed was poured through a HisTrap HP 5ml (GE Healthcare) column stabilized with wash buffer (pH 8.0, 50 mM Tris-HCl, 500 mM NaCl), and then elution buffer (pH 8.0, 50 mM Tris-HCl, 500 mM NaCl, 500 mM imidazole) was set to 0 to 100% gradient elution. As a result, it was found that the therapeutic protein CTP delta was isolated with imidazole at a concentration of 100 to 300 mM. The isolated therapeutic protein CTP delta was subjected to buffer exchange (pH 8.0, 20 mM Tris-HCl) in a cold room for 15 hours. After pouring the NaCl-removed CTP delta solution through the HiTrap Q HP 5ml (GE Healthcare) column stabilized with binding buffer (pH 8.0, 20 mM Tris-HCl), elution buffer (pH 8.0, 20 mM Tris-HCl, 1.0 M NaCl) was set from 0 to 100% gradient elution. As a result, it was found that the therapeutic protein CTP delta was isolated with NaCl at a concentration of 120 to 250 mM. Finally, therapeutic protein CTP delta having a dimer structure was purely isolated and purified using a HiLoad Superdex 75 16/600 column stabilized with pH 7.4, 10 mM phosphate and 150 mM NaCl. Quantification was performed at A₂₈₀ₙₘ using NanoDrop, and, as a result of calculating the purity using the ImageJ program, high purity (>96%) therapeutic protein CTP delta protein was obtained (FIG. 47).

### <Example 22> Mass spectrometry of coronavirus infectious disease COVID-19 therapeutic protein CTP delta

### 1. Mass spectrometry analysis

Mass spectrometry was used to identify the total mass and information on the amino acid sequence of the protein. Mass spectrometry of total proteins was performed using a high-performance matrix laser desorption ionization mass spectrometer (Bruker, UltrafleXtreme MALDI TOF/TOF). Hybrid triple quadrupole linear ion trap mass spectrometer (Sciex, QTRAP) and quadrupole orbitrap mass spectrometer (Thermo scientific, Q Exactive PLUS) were used for amino acid sequence analysis for the protein and micro liquid chromatography (Sciex, M5 MicroLC) was used for QTRAP, and nano-micro liquid chromatography (Waters, ACQUITY UPLC M-Class system) was used for Q Exactive PLUS.

### 2. Sample preparation for MALDI TOF and analysis of intact proteins using MALDI TOF

For total mass spectrometry of ACE2, RBD, and CTP delta proteins, 10 µg of protein obtained by removing low-molecular compounds using Microcon centrifugal filter (Merck, MRCPRT010) and sinapinic acid (Bruker, 8201345) matrix were mixed in a 1:1 ratio, and the mixture was then placed on a target plate for drying and subjected to the flexcontrol program of the high-performance matrix laser desorption ionization mass spectrometer. The mass value was normalized using the Starter kit for MALDI-TOF MS (Bruker, 8208241), and the spectrum was obtained with an average of 1000 laser shots based on the method provided by flexcontrol. Each spectral data was obtained using the flexanalysis program.

### 3. Sample preparation for LC-MS/MS analysis

For amino acid sequence analysis of a single protein, 20 µg of RBD, hACE2, and CTP delta proteins were mixed with 100 mM triethylammonium bicarbonate (Sigma, T7408-100ML) and 2% sodium dodecyl sulfate (Duksan, 6645) to a specific concentration. After adding 10 mM dithiothreitol (BIO-RAD, 161-0611) and treating at 56°C for 30 minutes, 20 mM iodoaetamide (Sigma, T1503) was added and shielded from light, followed by treatment at room temperature for 30 minutes. Then, using S-trap mini columns (PROTIFI, C02-mini-80), low molecular weight compounds were removed, and 2 µg of proteases including trypsin (Promega, V5111) and Glu-C (Promega, V1651) were added, followed by enzymatic degradation at 37°C for 16 hours. After termination of the reaction, isolation was performed with a centrifuge, drying was followed under vacuum, and 0.1% trifluoroacetic acid was added to prepare a sample. In addition, deglycosylation experiments were added for RBD and ACE2 proteins. For deglycosylation experiment, 100 mM triethylammonium bicarbonate and 2 µg of PNGase F (Promega, V4831) were added to the protease-treated sample, followed by enzymatic degradation at 37°C for 4 hours. After termination of the reaction, the proteins were isolated by centrifuge using S-trap mini columns. Vacuum drying was followed, and then 0.1% trifluoroacetic acid was added to prepare a sample. For the RBD and hACE2 proteins, a quadrupole orbitrap mass spectrometer was used, and experiments proceeded via nano-micro liquid chromatography. For the CTP delta protein, a hybrid triple quadrupole linear ion trap mass spectrometer was used, and experiments proceeded using micro-liquid chromatography.

### 4. LC-MS/MS Analysis: ACQUITY UPLC M-Class system - Q Exactive PLUS

All solvents used for liquid chromatography were of HPLC grade. Water (Duksan, 7732-18-5) and acetonitrile (Honeywell, AH015-4) were used as solvents, and dimethyl sulfoxide (Sigma, 472301-1L), trifluoroacetic acid (Thermo scientific, 28904), and formic acid (Thermo scientific, 28905) were used as a medium in the experimental solution. Nano-micro liquid chromatography was composed of solvent A (0.1% formic acid, 5% dimethyl sulfoxide in water) and solvent B (0.1% formic acid, 5% dimethyl sulfoxide in 80% acetonitrile). Acclaim^{™} PepMap^{™} 100 C18 LC Column (Thermo scientific, 164197) was used as a trapping column, and EASY-Spray^{™} HPLC Columns (Thermo scientific, ES803A) as an analytical column. Liquid chromatography was performed for 5 minutes in the presence of 95% solvent A and 5% solvent B at a flow rate of 8 µl/min in the trapping step after injecting 0.5 µg of the sample. In the analytical step, the flow rate was 250 nl/min. After 5 minutes under the conditions of 95% solvent A and 5% solvent B, the samples were analyzed by gradually increasing the rate of solvent B from 5% to 40% for 140 minutes, the column was activated for 15 minutes after increasing to 95% for 5 minutes again, and re-equilibration was followed by decreasing the rate of solvent B to 5% for 5 minutes and maintaining the state for 40 minutes. The mass values of quadrupole orbitrap mass spectrometer were calibrated by Pierce^{™} LTQ Velos ESI Positive Ion Calibration Solution (Thermo scientific, 88323). The analysis was conducted via full-MS and data dependent MS/MS methods. The calculated data was obtained using a proteome discoverer (Thermo scientific).

### 5. LC-MS/MS Analysis: M5 MicroLC - QTRAP

The column used for the analysis was a Kinetex 2.6u XB-C18 100A (Phenomenex, 00B-4496-AC). Micro liquid chromatography was composed of solvent A (0.1% formic acid in water) and solvent B (0.1% formic acid in acetonitrile). The liquid chromatography was performed at a flow rate of 20 µl/min after injecting 1.5 µg of the sample. After 5 minutes under the conditions of 95% solvent A and 5% solvent B, the samples were analyzed by gradually increasing the rate of solvent B from 5% to 40% for 45 minutes, the column was activated for 4 minutes after increasing to 95% for 5 minutes again, and re-equilibration was followed by decreasing the rate of solvent B to 5% for 3 minutes and maintaining the state for 3 minutes. The mass values of hybrid triple quadrupole linear ion trap mass spectrometer were calibrated by standard chemical kit (Sciex, 4406127). The analysis was conducted using Q3 MS and enhanced product ion method using information dependent acquisition. For the calculated data, the wiff file was converted to the mgf file using convert MS (proteowizard.sourceforge.net), and the results were obtained using searchGUI and peptide-shaker (compomics.github.io).

### 6. Results of intact protein mass spectrometry

The mass of the intact protein was measured using MALDI-TOF. The mass of RBD was 30 kDa. In addition to 30kDa, the peak was a dimer, appeared along with a 60kDa peak, a monomer with a charge (z) of 2 (m/z=15kDa), and a trimer with a charge (z) of 2 (m/z=45kDa). Considering that the mass of RBD calculated from the protein sequence is 25.9 kDa, it may be suggested that glycosylation caused the change in mass of 4.1 kDa. Similarly, the mass of hACE2 was measured to be about 102 kDa, and a dimer and a peak with a charge of 2 also appeared together. Considering that the mass of hACE2 calculated from the protein sequence is 84.4 kDa, it is suggested that the mass change of about 17.5 kDa was caused by glycosylation.

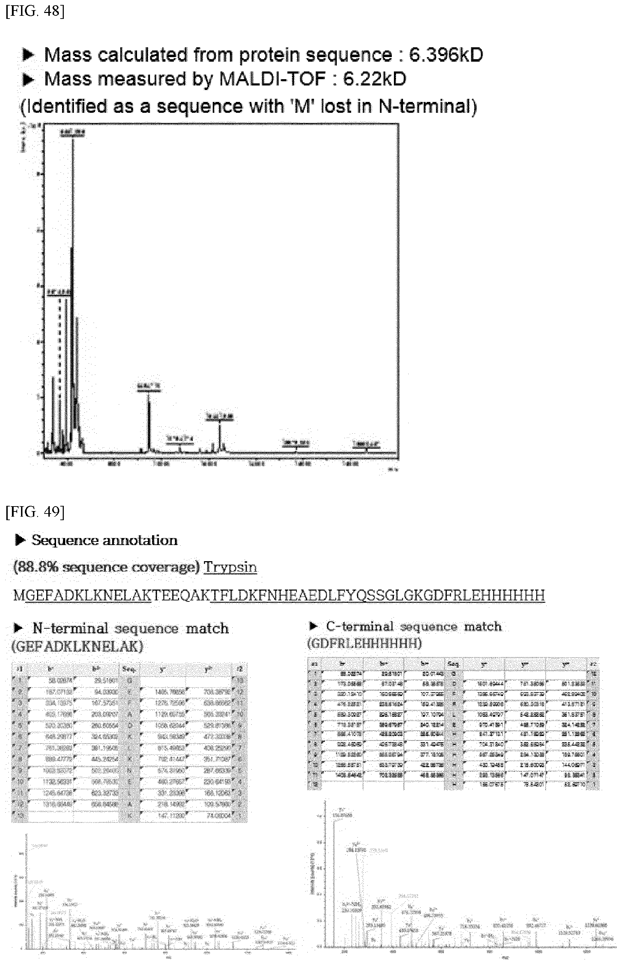

As a result of analysis of CTP-61, the mass calculated from the protein sequence was 6.396 kD, and that measured by MALDI-TOF was 6.221 kD, determining that it is a sequence with 'M' lost in the N-terminal (FIG. 48).

### 7. Identification of protein sequences

LC-MS/MS analysis was performed to identify the sequences of RBD, hACE2, and CTP delta. Each protein was subjected to protein analysis using proteases such as trypsin and Glu-C, wherein, for RBD and hACE2, N-glycosylated Asn was found by searching for amino acid residues in which Asn was changed to Asp by enzymatic activity of PNGase F.

The results of MS/MS analysis for CTP delta with N, C-terminus identified are shown in FIG. 49. Sequence analysis of the N, C-terminus is one of the means for identifying the state of the protein, and if the terminus of the protein is not identified, it may be considered as being degraded due to a problem in the preservation state of the protein. In the case of RBD and hACE2, the terminus of the proteins tends not to be identified (ID), so unless inaccuracy in the sequence or the effect of PTM matters, they are easily degraded, so care must be taken for preservation.

### <Example 23> Secondary structural stability and function/efficacy verification of coronavirus infectious disease COVID-19 therapeutic protein CTP delta

### 1. Circular dichroism (CD) experiment

Far UV circular dichroism was measured for RBD (PBS buffer), hACE2 (PBS buffer), and CTP delta. A JASCO-1500 machine was used with the protein concentration of 0.2 mg/ml and a quartz cell of 0.1 cm at a temperature of 20°C. Measurements were performed at a measurement speed of 20 nm/min, a bandwidth of 5 nm, and a digital integration time (D.I.T) of 4 seconds at a far UV wavelength of 190-250 nm. A graph was drawn by subtracting the data obtained by 5 times measurement for the protein by the data obtained by 5 times measurement for the buffer.

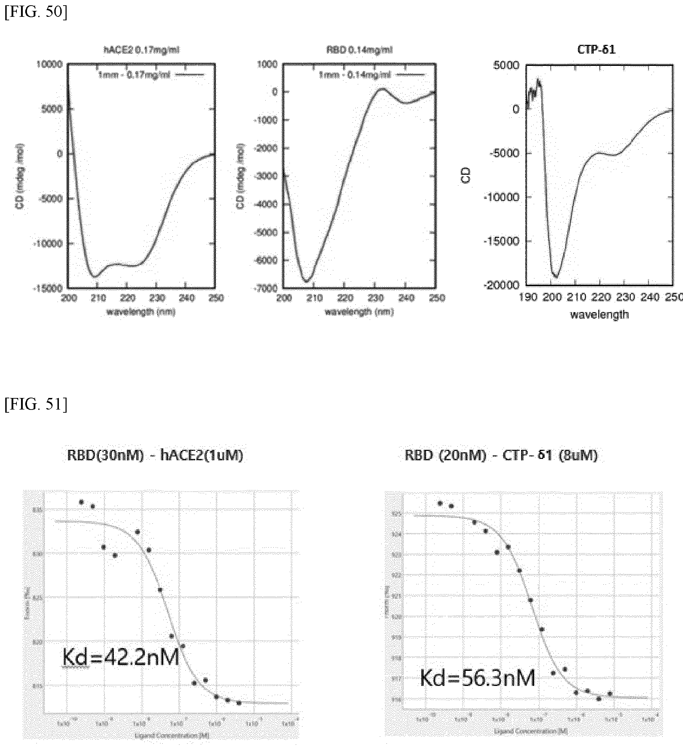

As a result, it was found that hACE2 is a protein having α-helix as a secondary structure, and RBD is a protein having a beta sheet as a secondary structure. It was found that CTP delta has both a helical structure and a coil structure (FIG. 50).

### 2. Microscale thermophoresis (MST) experiment

Fluorescence was attached to 100 µL of 8 µM RBD (PBS buffer with 0.05% Tween-20 added) using Monolith Protein Labeling Kit RED-NHS 2nd Generation. Ligand (hACE2 (PBS buffer) and CTP delta were prepared by 10 µL each with 16 concentrations from 16 µM to 0.488 nM using a 1:1 serial dilution. A mixed solution of 15 nM RBD and 8 µM-0.244 nM ligand was prepared by diluting the fluorescence-attached RBD to 30 nM and adding 10 µL of the RBD to each ligand of 16 concentrations. MST was measured 5 times for each ligand using a Monolith NT.115 machine with 60% excitation power and medium MST power. Kd (dissociation constant) value was calculated using Kdmodel of MO affinity analysis program provided by Monolith company. Experimental results having the median Kd value derived from 5 experiments are shown in a graph.

As a result, a change in concentration-dependent fluorescence intensity was observed, indicating that both hACE2 and CTP delta bind to RBD. The Kd values representing the binding strength were 56.3 nM for CTP-61 and 42.2 nM for hACE2 (FIG. 51). The Kd values of RBD and hACE2 are similar to that of 34.6 nM measured by using BLI (biolayer interferometry) and SPR (Surface Plasmon Resonance) in the Science paper (Wrapp et al, Science 367, 1260-1263 (2020)).

As the specific part of the present disclosure has been described in detail above, for those of ordinary skill in the art, it is clear that the specific description is only a preferred embodiment, and the scope of the present disclosure is not limited thereby. Accordingly, it is intended that the substantial scope of the present disclosure is defined by the appended claims and equivalents thereof.

## Claims

1. A protein, comprising any one amino acid sequence selected from amino acid sequences represented by SEQ ID NO: 1 to SEQ ID NO: 10 and specifically binding to a receptor binding domain (RBD) of coronavirus.

2. The protein of claim 1, wherein the protein inhibits binding between RBD of coronavirus and angiotensin-converting enzyme 2 (ACE2).

3. The protein of claim 1, wherein the coronavirus is SARS-CoV2.

4. The protein of claim 1, wherein the protein which comprises any one amino acid sequence selected from the amino acid sequences represented by SEQ ID NO: 1 to SEQ ID NO: 9 binds to D420 and K458 of SARS-CoV2 RBD, and the protein which comprises the amino acid sequence represented by SEQ ID NO: 10 binds to R454, K458, D467, and E471 of SARS-CoV2 RBD.

5. A polynucleotide encoding the protein of claim 1.

6. A recombinant vector, comprising the polynucleotide of claim 5.

7. A transformant transformed with the recombinant vector of claim 6 (except for humans).

8. A composition for diagnosing coronavirus infection, comprising the protein of any one of claims 1 to 4 as an active ingredient.

9. The composition of claim 8, wherein the coronavirus is SARS-CoV2.

10. A pharmaceutical composition for preventing or treating coronavirus infectious disease, comprising the protein of any one of claims 1 to 4 as an active ingredient.

11. The pharmaceutical composition of claim 10, wherein the coronavirus infectious disease is COVID-19.

12. A health functional food composition for preventing or ameliorating coronavirus infectious disease, comprising the protein of any one of claims 1 to 4 as an active ingredient.

13. A composition for drug delivery, comprising the protein of any one of claims 1 to 4 as an active ingredient.

14. The composition of claim 13, wherein the drug is an antiviral agent.

15. The composition of claim 13, wherein the antiviral agent is an antiviral agent against coronavirus.

16. The composition of claim 15, wherein the coronavirus is SARS-CoV2.
